(19) Europäisches Patentamt
European Patent Office
Office européen des brevets

(11) EP 2 628 737 A1

(12) **EUROPEAN PATENT APPLICATION**
published in accordance with Art. 153(4) EPC

(43) Date of publication:
**21.08.2013 Bulletin 2013/34**

(21) Application number: **11832175.1**

(22) Date of filing: **11.10.2011**

(51) Int Cl.:
***C07D 309/30*** *(2006.01)* ***A61K 31/366*** *(2006.01)*
***A61P 25/28*** *(2006.01)* ***A61P 25/08*** *(2006.01)*

(86) International application number:
**PCT/ES2011/070705**

(87) International publication number:
**WO 2012/049346 (19.04.2012 Gazette 2012/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.10.2010 ES 201001340**

(71) Applicant: **Neuron Biopharma, S.A.**
**18100 Armilla - Granada (ES)**

(72) Inventors:
- **BURGOS MUÑOZ, Javier Santos**
  **E-18100 Armilla - Granada (ES)**
- **RAMÍREZ MORENO, Carlos**
  **E-18100 Armilla - Granada (ES)**
- **SIERRA ÁVILA, Saleta**
  **E-18100 Armilla - Granada (ES)**
- **RAMOS MARTÍN, María del Carmen**
  **E-18100 Armilla - Granada (ES)**
- **ALFARO SÁNCHEZ, Juan María**
  **E-18100 Armilla - Granada (ES)**
- **ADRIO FONDEVILA, José Luis**
  **E-18100 Armilla - Granada (ES)**
- **VELASCO ÁLVAREZ, Javier**
  **E-18100 Armilla - Granada (ES)**

(74) Representative: **ABG Patentes, S.L.**
**Avenida de Burgos, 16D**
**Edificio Euromor**
**28036 Madrid (ES)**

(54) **HYPOCHOLESTEROLEMIC, ANTI-INFLAMMATORY AND ANTIEPILEPTIC NEUROPROTECTIVE COMPOUND**

(57) The present invention describes a compound of formula (I)

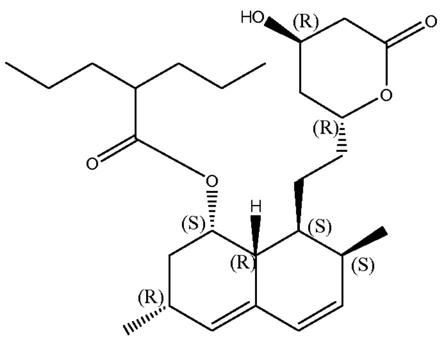

(I)

its hydroxy acid form, the pharmaceutically acceptable salts of said hydroxy acid and pharmaceutically acceptable prodrugs and solvates of the compound and of its hydroxy acid form and, in particular, said compound, its hydroxy acid form, salts, etc. for use in the prevention of: neurodegenerative diseases, cognitive impairment, diseases associated with undesired oxidation, age-associated pathological processes and progeria, cardiovascular diseases such as atherosclerosis, atrial fibrillation, dyslipidemia, hypercholesterolemia, hyperlipidemia, and hypertriglyceridemia, inflammation or inflammatory processes, or epilepsy, epileptic seizures and convulsions.

EP 2 628 737 A1

## Description

Field of the Invention

The present invention relates to the prevention and/or the treatment of neurodegenerative diseases or of diseases associated with undesired oxidation or of age-associated pathological processes, as well as to the decrease of cholesterol levels and to inhibition of the enzyme 3-hydroxy-3-methylglutaryl coenzyme A reductase for the prevention of dyslipidemia and of cardiovascular diseases, as well as of diseases associated with acute or chronic inflammatory processes, as well as to the prevention and/or the treatment of epilepsy, epileptic seizures or convulsions.

Background of the Invention

The high incidence of neurodegenerative diseases and of age-associated diseases is a problem of the first order worldwide. It is therefore necessary to search for neuroprotective compounds preventing or palliating said diseases. Of all of them, Alzheimer's disease (AD) is the most prevalent being estimated that 81 million people will suffer from this disease by 2040 (Blennow et al., Lancet 2006; 368: 387-403). It is estimated that half a million people are currently suffering from AD in Spain alone. The costs associated with this disease are proportionally high, and it is calculated that the total cost derived from caring for Alzheimer's patients is 81,000 and 22,000 million € in the United States and in the United Kingdom, respectively. Currently there are no effective drugs which prevent or impede this disease, therefore it is necessary to search for and validate novel neuroprotective compounds which prevent neuronal damage.

Different strategies are currently being followed for obtaining novel compounds since it has been seen that current drugs offer few benefits to the patients. These drugs temporarily delay (one year, at best) some symptoms of the illness but do not prevent their evolution. The current therapeutic options are based on inhibition of acetylcholinesterase with drugs such as donepezil, galantamine or rivastigmine, or on the capacity of memantine in antagonizing a glutamate receptor, NMDA (N-methyl-D-aspartic acid).

Due to the low success of these drugs, new lines of research have opened up and among them research on inhibitors of 3-hydroxy-3-methylglutaryl-coenzyme A reductase (HMGR) enzyme (known as statins) as therapeutic agents stands out in recent years. HMGR is the enzyme which catalyzes the limiting step in cholesterol biosynthesis, so its inhibition by statins is a common therapeutic strategy to reduce the high cholesterol levels associated with low density lipoproteins (LDL). These drugs reduce the risk of myocardial infarction and coronary death and are considered safe. Furthermore, hypercholesterolemia (defined as high blood cholesterol level) is the main risk factor for cardiovascular diseases (CVD), such as atherosclerosis.

Various genetic and environmental factors affecting cholesterol metabolism are associated with AD. For example, apolipoprotein E ε4 (apoE4) isoform is a risk factor for AD and is linked to an increase in cholesterol levels. Atherosclerosis, which has hypercholesterolemia as the main risk factor, also seems to be associated with AD. Furthermore, epidemiological studies indicate that high serum cholesterol levels increase the risk of AD, and it has been proposed that homeostatic regulation of cholesterol metabolism can be altered in Alzheimer's. On the other hand, a significant reduction of the risk of Alzheimer's in patients treated with statins has been described. All these studies jointly suggest that the cholesterol reduction levels can inhibit Alzheimer's disease pathogenesis (Cole & Vassar; Neurobiol Dis 2006; 22[2]:209-22).

Cholesterol is transported in the blood by means of different types of lipoproteins, in which the major cholesterol carriers are low density lipoprotein (LDL) and high density lipoprotein (HDL). LDLs are lipoproteins specialized in transporting cholesterol and triglycerides from the liver to peripheral tissues, where they are captured by the cells through LDL receptors (LDL-R) in the cell membrane. LDLs also regulate cholesterol synthesis, and high LDL cholesterol levels have been associated with the risk of suffering from CVD. In turn, HDLs are lipoproteins which transport cholesterol from the different tissues to the liver. Due to the fact that HDLs can remove cholesterol from arteries and transport it back to the liver for excretion, they are given a protective role against cardiovascular diseases. HMGR inhibitors are the most successful hypolipidemic agents throughout history, being capable of reducing total cholesterol levels based on decreasing LDL cholesterol levels without altering HDL cholesterol levels.

New properties of statins have recently been described, especially at the level of brain damage caused by trauma or in dementias, new activities have been proposed (Pahan, Cell Mol Life Sci. 2006; 63[10]: 1165-78), and certain statins (e.g., simvastatin) have been demonstrated to intensify learning and memory capacity in mice (Ling et al., Ann Neurol. 2006; 60[6]: 729-39) or protect against convulsive seizures associated with epileptic phenomena (Lee et al., Neurosci Lett. 2008; 440: 260-4). Furthermore, statins have also demonstrated their effectiveness in phase II clinical trials which suggest positive results with respect to the treatment of cerebral vasospasm (Fandino et al., Neurocirugia. 2007; 18: 16-27), as well as against neuronal death induced by ischemic damage in the retina (Honjo et al., Arch. Ophthalmol. 2002; 120: 1707-13). Nevertheless, it is currently being discussed whether the neuroprotective effects of different commercial statins (e.g., atorvastatin, lovastatin, simvastatin, etc.) are due to a direct effect on lipid metabolism

or, in contrast, whether they are a result of alternative routes.

In addition and in relation to the continuous search for causal events of both NDDs and CVDs, chronic inflammation has been proposed as an underlying phenomenon to such diseases. For example, it has been found that low-grade peripheral systemic inflammation is associated with the increase of cognitive damage. In this sense it has been determined that systemic inflammation is a risk factor for suffering AD (reviewed in: Holmes et al., "Systemic inflammation and disease progression in Alzheimer disease" Neurology. 2009).

Brief Description of the Invention

Although there is literature relating to the potential neuroprotective effect of statins, the authors of this invention have found that a derivative of a non-commercial monacolin J, specifically 2-propylpentanoic acid (1S,3R,7S,8S,8aR)-1,2,3,7,8,8a-hexahydro-3,7-dimethyl-8-[2-[(2R,4R)-tetrahydro-4-hydroxy-6-oxo-2H-pyran-2yl]ethyl]-1-naphthalenyl ester (also sometimes identified as NST0060 in this patent application) is an agent with a surprising neuroprotective potential, in addition to having a high capacity of crossing the blood-brain barrier (BBB) and high efficiency in reducing cholesterol levels, specifically inhibiting HMGR, as well as protecting against epilepsy, epileptic seizures and convulsions. Furthermore, and surprisingly, this compound is safer than commercial statins, showing toxicity levels under the levels of the statin showing the highest level of biosafety, simvastatin. Additionally, it is a compound that is less expensive to synthesize due to the low cost of the side chain to be added to the monacolin J molecule.

The neuroprotective activity of said compound has been clearly shown against different aggressions which cause neuronal death in human cell lines of cholinergic origin by means of different types of aggressions which cause oxidative stress, inhibition of protein phosphatase-1, inhibition of succinate dehydrogenase, endoplasmic reticulum stress and apoptosis (Example 2, Figures 1 to 6). Said example clearly show the potential use of said compound in the prevention and/or treatment of neuronal death associated with neurodegenerative diseases (e.g., Alzheimer's, Parkinson's, multiple sclerosis, amyotrophic lateral sclerosis, *status epilepticus,* Huntington's, etc.) or of diseases associated with undesired oxidation or of age-associated pathological processes.

To study the mechanism of action of this compound and to confirm its neuroprotective effect, its modulation on the seladin-1/DHCR24 gene expression, a gene involved in AD (Example 3, Figure 7), has been studied, observing that compound NST0060 is capable of increasing the mRNA levels of this neuroprotective gene in a dose-dependent manner in human cell lines of cholinergic origin.

For the purpose of better defining the blood-brain barrier crossing of compound NST0060, the inventors analyzed different parameters such as theoretical lipophilicity, the percentage of crossing and the effective permeability (Figure 8 and Table 1) as described in Example 4, determining that NST0060 has a high BBB crossing, very close to the positive control used (verapamil).

The hypolipidemic activity of said compound has been clearly shown by means of determining inhibition of HMGR in comparison to a marketed statin, simvastatin (Example 5, Figure 9). For the purpose of better defining the hypocholesterolemic effect of the compound, the inventors analyzed the hypocholesterolemic activity with greater detail by means of analyzing cholesterol reductions in a human hepatic cell line (Figure 10) as described in Example 5. Said example clearly shows the potential use of said compound in the prevention and/or treatment of hypercholesterolemia associated with cardiovascular diseases (e.g., myocardial infarction, atherosclerosis, congenital cardiopathy, acquired cardiopathy, ischemic cardiopathy, hypertensive cardiopathy, valvulopathies, cardiomyopathies, blood disorders, etc.).

The biosafety of compound NST0060 has been clearly shown by means of evaluating its toxicity in a zebrafish embryo model in comparison with a marketed statin, simvastatin, observing that NST0060 is less toxic than simvastatin at different concentrations since it produces lower mortality (Example 6, Table 2), a higher percentage of healthy larvae at the end of the experiment (Example 6, Figure 11), and less disruption of the heart rate than simvastatin (Example 6, Figures 12).

The anti-inflammatory activity of said compound has been clearly shown in a model of systemic inflammation in mice generated by means of the intraperitoneal injection of lipopolysaccharide (LPS) (Example 7, Figures 13 to 17). Said example clearly shows the potential use of said compound in the prevention and/or treatment of inflammation and associated diseases, among which neurodegenerative diseases or cardiovascular diseases are found.

The antiepileptic and anticonvulsant activity of said compound has been clearly shown by means of determining the protection against epileptic seizures and convulsions in a model of epilepsy in mice (Example 8, Figures 18 and 19). Said example clearly shows the potential use of this compound in the prevention and/or treatment of epilepsy and of convulsive seizures or convulsions.

The *in vivo* antioxidant activity of said compound has been clearly shown by means of determining the protection against oxidative stress caused in the brain of mice inoculated with an excitotoxic substance (Example 9, Figures 20 and 21). Said example clearly shows the potential use of this compound in the prevention and/or treatment of diseases associated with undesired oxidation.

The protective effect of NST0060 has been clearly shown by means of determining levels of survival after an

excitotoxic damage causing death in mice (Example 10, Figure 22). Said example clearly shows the potential use of this compound in the prevention and/or treatment of diseases associated with excitotoxic syndrome, such as neurodegenerative diseases.

Therefore, one aspect of the present invention relates to a compound of formula (I) [also sometimes identified in this patent application as NST0060]:

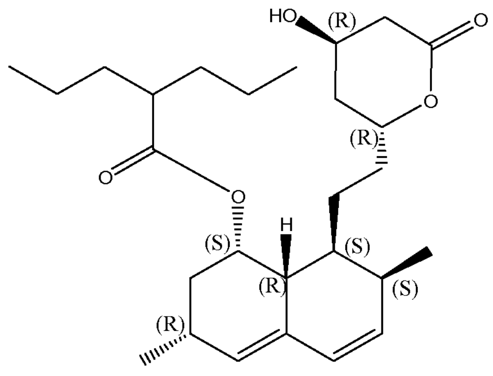

(I)

to its hydroxy acid form, to the pharmaceutically acceptable salts of said hydroxy acid and to pharmaceutically acceptable prodrugs and solvates of the compound and of its hydroxy acid form.

Another aspect of the present invention is a pharmaceutical composition comprising a compound of formula (I) and/or its hydroxy acid form and/or a pharmaceutically acceptable salt of said hydroxy acid and/or a pharmaceutically acceptable prodrug or solvate of the compound or of its hydroxy acid form, and at least one pharmaceutically acceptable adjuvant, carrier and/or vehicle.

Another aspect of the present invention relates to a compound of formula (I), to its hydroxy acid form or a pharmaceutically acceptable salt of said hydroxy acid and/or to a pharmaceutically acceptable prodrug or solvate of the compound or of its hydroxy acid form for use as a medicinal product.

According to another aspect, the present invention relates to a compound of formula (I), to its hydroxy acid form or to a pharmaceutically acceptable salt of said hydroxy acid and/or to a pharmaceutically acceptable prodrug or solvate of the compound or of its hydroxy acid form for use as a neuroprotective agent, particularly in the prevention and/or the treatment of:

a. neurodegenerative diseases (e.g., Alzheimer's, Parkinson's, multiple sclerosis, amyotrophic lateral sclerosis, *status epilepticus*, Huntington's, etc.), more specifically, as a neuroprotective agent against oxidative stress, inhibition of protein phosphatase-1, inhibition of succinate dehydrogenase, endoplasmic reticulum stress and apoptotic processes associated with said chronic neurodegenerative diseases,
b. cognitive impairment,
c. diseases associated with undesired oxidation,
d. age-associated pathological processes and progeria,
e. cardiovascular diseases such as atherosclerosis, atrial fibrillation, dyslipidemia, hypercholesterolemia, hyperlipidemia, and hypertriglyceridemia, or
f. inflammation, inflammatory processes and diseases presenting with or caused by inflammation
g. epilepsy, epileptic seizures and convulsions

One aspect of the present invention relates to the use of a compound of formula (I), of its hydroxy acid form, of a pharmaceutically acceptable salt of said hydroxy acid and/or of a pharmaceutically acceptable prodrug or solvate of the compound or of its hydroxy acid form in the manufacture of a medicament. According to a particular embodiment, the medicament is for being used as a neuroprotective agent, particularly in the prevention and/or the treatment of:

a. neurodegenerative diseases (e.g., Alzheimer's, Parkinson's, multiple sclerosis, amyotrophic lateral sclerosis, *status epilepticus*, Huntington's, etc.), more specifically, as a neuroprotective agent against oxidative stress, inhibition of protein phosphatase-1, inhibition of succinate dehydrogenase, endoplasmic reticulum stress and apoptotic processes associated with said chronic neurodegenerative diseases,
b. cognitive impairment,

c. diseases associated with undesired oxidation,
d. age-associated pathological processes and progeria,
e. cardiovascular diseases such as atherosclerosis, atrial fibrillation, dyslipidemia, hypercholesterolemia, hyperlipidemia, and hypertriglyceridemia, or
f. inflammation, inflammatory processes and diseases characterized or caused by inflammation
g. epilepsy, epileptic seizures and convulsions

Another aspect of the present invention is a compound of formula (I), its hydroxy acid form or a pharmaceutically acceptable salt of said hydroxy acid and/or a pharmaceutically acceptable prodrug or solvate of the compound or of its hydroxy acid form for use in increasing seladin-1/DHCR24 gene expression.
Another aspect of the present invention is a compound of formula (I), its hydroxy acid form or a pharmaceutically acceptable salt of said hydroxy acid and/or a pharmaceutically acceptable prodrug or solvate of the compound or of its hydroxy acid form for use in the prevention and/or treatment of diseases related to the seladin-1/DHCR24 gene.
Another aspect of the present invention relates to the use of a compound of formula (I), of its hydroxy acid form, of a pharmaceutically acceptable salt of said hydroxy acid and/or of a pharmaceutically acceptable prodrug or solvate of the compound or of its hydroxy acid form in the in the manufacture of a medicament characterized by increasing seladin-1/DHCR24 gene expression.
In another aspect, the invention relates to a method for the prevention and/or treatment of neurodegenerative diseases, cognitive impairment, diseases associated with undesired oxidation, age-associated pathological processes and progeria, cardiovascular diseases such as atherosclerosis, atrial fibrillation, dyslipidemia, hypercholesterolemia, hyperlipidemia and hypertriglyceridemia, epilepsy, epileptic seizures and convulsions, or diseases characterized or caused by inflammation, in a subject in need of treatment, comprising administering to said subject a therapeutically effective amount of a compound of formula (I), its hydroxy acid form or a pharmaceutically acceptable salt of said hydroxy acid and/or a pharmaceutically acceptable prodrug or solvate of the compound or of its hydroxy acid form.

Brief Description of the Figures

Figure 1 is an XY scatter chart depicting the protective effect of compound NST0060 against death caused by xanthine/xanthine oxidase (XXO). The figure shows the percentage of cell death (taking as 100% the cell death caused by XXO) of the cultures treated with 10 μM xanthine (X) 60 mU/mL xanthine oxidase (XO) and NST0060 at different concentrations, representing the mean+SD of 3 independent experiments in triplicate. * Significant difference with respect to the treatments with XXO alone according to the Student's t test ($p<0.05$).
Figure 2 is an XY scatter chart depicting the protective effect of compound NST0060 against death caused by okadaic acid (OA). The figure shows the percentage of cell death (taking as 100% the cell death caused by OA) of the cultures treated with 20 nM OA and NST0060 at different concentrations, representing the mean+SD of 2 independent experiments in triplicate. * Significant difference with respect to the treatments with OA alone according to the Student's t test ($p<0.05$).
Figure 3 is an XY scatter chart depicting the protective effect of compound NST0060 against death caused by 3-nitropropionic acid (3-NP). The figure shows the percentage of cell death (taking as 100% the cell death caused by 3-NP) of the cultures treated with 30 μM 3-NP and NST0037 at different concentrations, representing the mean+SD of 2 independent experiments in triplicate. * Significant difference with respect to the treatments with 3-NP alone according to the Student's t test ($p<0.05$).
Figure 4 is an XY scatter chart depicting the protective effect of compound NST0060 against death caused by tunicamycin (TM). The figure shows the percentage of cell death (taking as 100% the cell death caused by TM) of the cultures treated with 24 μM TM and NST0060 at different concentrations, representing the mean+SD of 2 independent experiments in triplicate. * Significant difference with respect to the treatments with TM alone according to the Student's t test ($p<0.05$).
Figure 5 is a bar graph depicting the inhibitory effect of the pretreatment of compound NST0060 on the activation of caspase 3/7 (apoptosis effector caspases), and where apoptosis is caused by camptothecin (CPT). The figure shows the percentage of active caspase 3/7 in reference to the control cells without CPT and with 50 μM of CPT, and the effect of the pretreatment with NST0060 at 10 or 40 μM or of the inhibitor Z-VAD-fmk at 50 μM (control for inhibition), representing the means±SD of 2 independent experiments in triplicate. * Significant difference with respect to the treatment with CPT according to the Student's t test ($p<0.05$).
Figure 6 is a bar graph depicting the inhibitory effect of the pretreatment of compound NST0060 on DNA fragmentation caused by CPT. The figure shows the percentage of DNA fragmentation in reference to the control cells without CPT and with 50 μM of CPT, and the effect of the pretreatment with NST0060 at 10 or 40 μM or of the inhibitor Z-VAD-fmk at 50 μM (control for inhibition), representing the means±SD of 2 independent experiments in duplicate. * Significant difference with respect to the treatment with CPT according to the Student's t test ($p<0.05$).

Figure 7 is a bar graph depicting GAPDH-normalized seladin-1/DHCR24 gene expression after cell treatments with compound NST0060 at 1, 4, 10 and 40 μM, quantified by means of quantitative RT-PCR. The means±SD of an experiment in triplicate are represented, and the values of two controls (untreated cells) in the assay are shown. * Significant difference with respect to the controls according to the Student's t test ($p<0.05$).

Figure 8 is an XY scatter chart depicting the effective permeability expressed as $P_e$ (cm/s) with respect to the BBB crossing (%) of simvastatin and NST0060 in their active forms; both parameters have been determined *in vitro* by means of the PAMPA method. Verapamil and theophylline were used as positive and negative controls, respectively.

Figure 9 is an XY scatter chart depicting the percentage of HMGR activity with respect to the control exerted by simvastatin and NST0060 in their active forms at different concentrations determined by means of an *in vitro* assay. The results are the mean±SD of the five independent assays in duplicate that were carried out.

Figure 10 is a bar graph depicting the effect of simvastatin and of NST0060 in their active forms on total cholesterol levels in the human cell line HepG2. The results are expressed as the percentage of cholesterol reduction with respect to the control in each line after incubating the compounds for 20 hours in the absence of FBS. The determinations were carried out by enzymatic and fluorometric methods, and the results are shown as the mean±SD of 2 independent assays in triplicate. * Significant difference with respect to the untreated cells according to the Student's t test ($p<0.05$).

Figure 11 is a bar graph depicting the percentage of healthy larvae after exposure to compound NST0060 in comparison with simvastatin. The results are expressed as the means±SD of the percentage of healthy animals (without toxicological problems) after treatment with different doses of NST0060 and simvastatin. The black bars represent the group of animals treated with NST0060 and the gray bars represent the animals treated with simvastatin.

Figure 12 is a bar graph depicting the variation of cardiotoxicity of compound NST0060 in comparison with simvastatin according to dose. The results are expressed as the means±SD of the percentage of the heart rate of the embryos or larvae treated with increasing doses of NST0060 or of simvastatin at 72 hours post-treatment. The black horizontal dotted line represents the mean value of the heart rate corresponding to the controls. * Significant difference of treatments with respect to the control according to the Student's t test ($p<0.05$). # Significant difference between NST0060 and simvastatin according to the Student's t test ($p < 0.05$).

Figure 13 is a bar graph depicting the anti-inflammatory effect of simvastatin and of NST0060 mediated by the decrease of the plasma concentration of TNFα in mice 2 hours after the i.p. administration of LPS at 8 mg/kg. Treatments were administered 12 hours and 1 hour before injecting LPS in male C57BL6 mice (n=8). The results are expressed as plasma concentration of TNFα in pg/mL determined by means of ELISA and are the mean±SEM. * Significant difference with respect to the control group (vehicle) according to the Student's t test ($p<0.05$); # significant difference between treatments according to the Student's t test ($p<0.05$).

Figure 14 is a Kaplan-Meier graph depicting the survival rate of male C57BL6 mice after the i.p. injection of physiological saline or LPS 8 mg/kg after i.p. treatment with NST0060 at 50 mg/kg or vehicle. Treatments were administered 12 hours and 1 hour before injecting LPS (n=8/group). Vehicle was administered with the same regimen (n=8). * Significant difference with respect to the control group according to the Student's t test ($p<0.05$).

Figure 15 is an XY scatter chart depicting the score associated with the symptomatology of the animals inoculated i.p. with LPS 8 mg/kg after treatment with NST0060 at 50 mg/kg over time. Treatments were administered 12 hours and 1 hour before injecting LPS in male C57BL6 mice (n=8/group). * Significant difference with respect to the control group+LPS according to the Student's t test ($p<0.05$). The different steps of the score correspond to: 0 = asymptomatic; 1 = mild lethargy and hypothermia; 2 = severe lethargy, diarrhea and tremors; 3 = absolute immobility.

Figure 16 is a bar graph depicting the anti-inflammatory effect of NST0060 mediated by the decrease of the plasma concentration of TNFα in mice 2 hours or 4 hours after the i.p. administration of LPS at 8 mg/kg. Treatments were administered 12 hours and 1 hour before injecting LPS in male C57BL6 mice (n=8 to 16). The results are expressed as the plasma concentration of TNFα in pg/mL determined by means of ELISA and are the mean±SEM. * Significant difference with respect to the control group (vehicle) according to the Student's t test ($p<0.05$); # significant difference between treatments according to the Student's t test ($p<0.05$).

Figure 17 is a bar graph depicting the anti-inflammatory effect of NST0060 mediated by the decrease of the plasma concentration of IL-1β in mice 2 hours after the i.p. administration of LPS at 8 mg/kg. Treatments were administered 12 hours and 1 hour before injecting LPS in male C57BL6 mice (n=8). The results are expressed as the plasma concentration of IL-1β in ng/mL determined by means of ELISA and are the mean±SEM. * Significant difference with respect to the control group (vehicle) according to the Student's t test ($p<0.05$).

Figure 18 is a bar graph depicting the mean±SEM of the time in minutes after the inoculation of KA when the first convulsion occurs (y axis) according to the pretreatment received (x axis). The pretreatment group pretreated with the vehicle is depicted in black and the treatment group treated with NST0060 at 50 mg/Kg by weight is depicted in white. * p-value<0.05 according to the Student's t test with two-tailed distribution for samples with unequal variance.

Figure 19 is an XY scatter chart depicting the level of epileptogenic symptom severity according to the Racine scale (Racine, Electroencephalogr Clin Neurophysiol 1972; 32[3]: 281-94) over time post-inoculation of the epileptogenic substance (kainic acid or kainate or KA). The graph shows the progression of the epileptogenic state of the animals according to the treatment received: vehicle is depicted with squares and a solid line and the treatment group treated with NST0060 at 50 mg/Kg by weight is depicted with circles and dotted lines. *p-value<0.05 according to the general analysis of variance by means of an ANOVA test and a Newman-Keuls post-hoc test.

Figure 20 is a panel with representative images of the photon emission kinetics of the different treatment groups: (i) vehicle+PBS, (ii) vehicle+KA and (iii) NST0060+KA. A color scale is shown next to the images that were taken depicting the intensity of the signal from 6000 to 30000 photons/sec/$cm^2$/sr.

Figure 21 is an XY scatter chart with the mean±SEM of the quantification of the signal at the different points in time of the subjects in the experimental groups: (i) vehicle+PBS depicted with triangles and dotted lines, (ii) vehicle+KA depicted with squares and a solid line and (iii) NST0060+KA depicted with circles and dotted lines. * p-value<0.05 for each time point with respect to the vehicle+PBS group according to the Student's t test with one-tailed distribution for samples with unequal variance.

Figure 22 is a Kaplan-Meier graph depicting the survival rate of the mice according to treatment (-24 and -0.5 hours with respect to inoculation of the excitotoxic substance [KA or Kainate]), inoculation of the excitotoxic substance (0 days post-inoculation [d.p.i.]) and subsequent treatment (up to 7 d.p.i.).

## Detailed Description of the Invention

### Definitions

To aid in understanding the invention object of this patent application, the meaning of some terms and expressions used in the context of the invention is explained below.

As it is used herein, the term "neuroprotective" refers to any substance capable of causing the attenuation or disappearance of the effects of neuronal degeneration or death by means of any mechanism known or to be known, for example, necrosis, apoptosis, autophagia, oxidative damage, excitotoxicity, endoplasmic reticulum damage, deposition of byproducts, cytoskeleton disorganization, inhibition of the electron transport chain, loss of cell architecture, etc., or to the reduction or disappearance of the side effects thereof.

As it is used herein, the term "statin" refers to an inhibitor of the 3-hydroxy-3-methylglutaryl-coenzyme A reductase (HMGR) enzyme, which catalyzes the limiting step of cholesterol biosynthesis and includes any natural, synthetic or semisynthetic statin. Some statins can be in the closed form (lactone) or in the open form (hydroxy acid). Hydroxy acids (open form) can be prepared from the corresponding lactones by conventional hydrolysis, for example, with sodium hydroxide in methanol, sodium hydroxide in tetrahydrofuran-water and the like. In the open form (hydroxy acid), the statins react to form salts with pharmaceutically acceptable metal and amine cations formed from organic or inorganic bases. The pharmaceutically acceptable salts of the statins can differ from the corresponding free acids in some physical characteristics such as solubility and melting point, but they are considered equivalent to the free acid form for the purposes of this invention. The free open form (hydroxy acid) of statins can be regenerated from the salt form, if desired, by contacting the salt with a diluted aqueous solution of an acid such as hydrochloric acid and the like. The closed form (lactone) of statins can be regenerated by dissolving the open form (hydroxy acid) in an inert solvent such as, for example, toluene, benzene, ethyl acetate and the like, at temperatures comprised between approximately 0°C and approximately the boiling point of the solvent, typically (although not necessarily) with simultaneous separation of the resulting water and catalysis with strong acids, e.g., hydrochloric acid and the like. Likewise, the statins can exist in a solvated or non-solvated form and such forms are equivalent to the non-solvated form for the purposes of this invention.

As it is used herein, the term "cardioprotective" refers to any substance capable of causing the attenuation or disappearance of the underlying effects of cardiovascular diseases or cardiopathies or of cardiac damage by means of any mechanism known or to be known, for example, necrosis, apoptosis, ischemia, arrhythmia, deposition of byproducts, loss of cell architecture, etc., or to the reduction or disappearance of the side effects thereof.

As it is used herein, the term "hypolipidemic" refers to any pharmacologically active substance having the property of reducing blood lipid levels or lipid levels in other tissues. The importance of these substances is due to the fact that the excess of some types of lipids (cholesterol or triglycerides) or lipoproteins is one of the main risk factors for cardiovascular diseases.

As it is used herein, the term "hypocholesterolemic" refers to any pharmacologically active substance having the property of reducing blood cholesterol levels or cholesterol levels in other tissues.

As it is used herein, the term "anti-inflammatory" refers to the attenuation of inflammatory processes or to acute inflammation or to chronic or systemic inflammation, for example in length and/or in intensity, or to the disappearance of inflammatory processes or to acute inflammation or to chronic or systemic inflammation, or to the reduction or disappearance of the side effects thereof.

As it is used herein, the term "biosafe" refers to the absence of toxic effects, generation of tumors, embryonic development disorders (teratogenesis) or other adverse effects.

As it is used herein, the term "neurodegenerative disease" includes diseases which result from the degeneration or deterioration of nervous tissue, particularly of neurons, leading over time to a dysfunction or to a disability; the term degeneration includes loss of cell viability, loss of cell function and/or loss of the number of cells (neurons or others). Illustrative, non-limiting examples of neurodegenerative diseases include Alzheimer's disease, Huntington's disease, Parkinson's disease, amyotrophic lateral sclerosis (ALS), multiple sclerosis, etc. In a particular embodiment, said neurodegenerative disease is a disease related to neuronal death caused by a substance which, for example, causes oxidative stress or endoplasmic reticulum stress or apoptosis or excitotoxicity or cytoskeleton disorganization or inhibition of the electron transport chain or neuronal death in general.

As it is used herein, the term "disease associated with undesired oxidation" refers to a disease caused by undesired oxidation (e.g., excessive oxidation) or in which said undesired oxidation is a symptom. Said undesired oxidation can be a result of the damage caused by free radicals on proteins, DNA and/or lipids independently from the specific free radical involved or from the target. Undesired oxidation involves an excessive generation of free radicals which can cause a dysfunction in cells, tissues or organs and can therefore form a potential mechanism of a disease. In a particular embodiment, said undesired oxidation can be caused by age (aging) or by a neurodegenerative process and can cause by itself or in combination with other factors the onset of several diseases. In a specific embodiment, said undesired oxidation relates to the oxidative damage caused by a substance which causes oxidative stress.

As it is used herein, the term "age-associated pathological process" refers to any age-related event or combination of events causing loss of cell viability of the nervous tissue or cell sensitization of the nervous tissue, loss of cell function and/or loss of the number of cells (neurons or others), including cell metabolic dysfunction, stress processes, infections by pathogens, genetic alterations, genetic susceptibility, trauma, ischemia, epilepsy, etc.

As it is used herein, the term "cardiovascular disease" refers to any disease or dysfunction or alteration of the heart or of the rest of the cardiovascular system or of the blood.

As it is used herein, the term "inflammation" refers to a non-specific response to environmental aggressions, or a response generated by inflammatory agents (bacteria, viruses, parasites, fungi, cell death processes such as apoptosis or necrosis, molecules activating the inflammatory response such as uric acid, nucleotides, nucleic acids, toxins..., traumas, foreign bodies, physical agents, vascular disorders, degenerative processes, immune and autoimmune disorders, hypersensitivity, etc.

As it is used herein, the term "cognitive impairment" refers to the loss or alteration of mental functions, such as memory, orientation, language, visual recognition or conduct, which interfere with the social activity and interaction of the person affected persistently over time.

As it is used herein, the term "epilepsy" refers to a chronic brain syndrome having various causes, characterized by recurrent seizures due to excessive hypersynchronic discharges of nervous impulses by brain neurons, associated eventually with various clinical and paraclinical manifestations. The seizures can be convulsive or non-convulsive. Epilepsy can have many causes; in some cases it can be due to different types of brain injuries (e.g., brain traumas, sequelae of meningitis, tumors, etc.); in other cases there is no injury but a genetic predisposition to seizures; in other cases, the etiology of the epilepsy can be environmental, due to pharmacological treatments, due to excitotoxicity, trauma, stress processes, aging, development problems, neurological diseases, psychological crises, problems during gestation, problems during labor, etc.

As it is used herein, the term "epileptic or convulsant" refers to any epileptic seizure or convulsion of any etiology, for example, genetic, environmental, due to pharmacological treatments, due to excitotoxicity, due to trauma, due to stress processes, due to aging, due to development problems, due to neurological diseases, due to psychological crises, due to problems during gestation, due to problems during labor, etc. An epileptic seizure occurs when an abnormal electrical activity in the brain causes an involuntary change of body movement or function, feeling, in the capacity of being alert or in behavior, and can be partial or generalized (convulsive or non-convulsive).

As it is used herein, the term "subject" refers to a member of a mammal species and includes but is not limited to domestic animals, primates and humans; preferably, the subject is a male or female human being of any age or race. In a particular embodiment, said subject is a mammal which suffers, or is susceptible to suffering, age-associated pathological processes, such as aging, or a neurodegenerative disease, such as a chronic neurodegenerative disease.

As it is used herein, the term "pharmaceutically acceptable" refers to the fact that the compound is physiologically tolerable and generally does not cause an allergic reaction or a similar unfavorable reaction, such as a gastric disorder, dizziness or the like, when administered to a subject; said term "pharmaceutically acceptable" preferably means approved by a government regulatory agency or listed in the United States Pharmacopoeia or in another generally recognized pharmacopoeia for use in animals (e.g., European Pharmacopoeia, etc.).

As it is used herein, the term "pharmaceutically acceptable salt" includes "pharmaceutically acceptable metal salts" as well as "pharmaceutically acceptable amine salts". The term "pharmaceutically acceptable metal salt" contemplates salts formed with sodium, potassium, calcium, magnesium, aluminum, iron or zinc ions. The term "pharmaceutically

acceptable amine salt" contemplates salts with ammonia and organic nitrogen bases strong enough to form salts with carboxylic acids. Said pharmaceutically acceptable salts can be obtained by conventional methods known by persons skilled in the art.

The compound 2-propylpentanoic acid (1S,3R,7S,8S,8aR)- 1,2,3,7,8,8a-hexahydro-3,7-dimethyl-8-[2-[(2R, 4R)-tetrahydro-4-hydroxy-6-oxo-2H-pyran-2yl]ethyl]-1-naphthalenyl ester can be obtained by semi-synthesis methods, such as those described for other compounds of the same family in United States patent US 4866090.

A number of assays performed by the inventors have clearly shown the neuroprotective effect of compound NST0060 against the action of a substance causing oxidative stress, as well as its neuroprotective effect against a substance causing cytoskeleton disorganization, as well as its neuroprotective effect against a substance causing inhibition of the electron transport chain, as well as its neuroprotective effect against the action of a substance causing endoplasmic reticulum stress, and its neuroprotective effect against the action of a substance causing apoptosis in human cholinergic neurons.

The neuroprotective effect against the action of a substance causing oxidative stress, such as xanthine/xanthine oxidase (XXO), is described in Example 2. It is observed in said example that compound NST0060 is capable of significantly and quantitatively reducing neuronal death caused by oxidative stress, which clearly shows the neuroprotective capacity of this compound (Figure 1). For the purpose of better defining the neuroprotective effect of the compound, the inventors analyzed the neurodegenerative process with greater detail by means of analyzing neuronal death caused by the action of okadaic acid (OA) which causes death by cytoskeleton disorganization by means of the inhibition of protein phosphatase-1, determining that compound NST0060 is capable of quantitatively and significantly reducing neuronal death caused by cytoskeleton disorganization, which clearly shows the neuroprotective capacity of said compound (Figure 2). For the purpose of better defining the neuroprotective effect of the compound, the inventors analyzed the neurodegenerative process with greater detail by means of analyzing neuronal death caused by the action of 3-nitropropionic acid (3-NP) which causes death by means of the inhibition of the electron transport chain by means of inhibition of succinate dehydrogenase, and it is accepted as a model of Huntington's disease, determining that compound NST0060 is capable of quantitatively and significantly reducing neuronal death caused by inhibition of the electron transport chain, which clearly shows the neuroprotective capacity of said compound (Figure 3). For the purpose of better defining the neuroprotective effect of the compound, the inventors analyzed the neurodegenerative process with greater detail by means of analyzing neuronal death caused by the action of a substance causing endoplasmic reticulum stress (tunicamycin), determining that compound NST0060 is capable of significantly and quantitatively reducing neuronal death caused by endoplasmic reticulum stress, which clearly shows the neuroprotective capacity of said compound (Figure 4). For the purpose of better defining the neuroprotective effect of the compound, the inventors analyzed the neurodegenerative process with greater detail by means of analyzing neuronal death caused by the action of a substance causing apoptosis (camptothecin, CPT), determining that compound NST0060 is capable of significantly and quantitatively reducing the activation of effector caspases 3/7 causing apoptosis, which clearly shows the neuroprotective capacity of said compound (Figure 5). Likewise, for the purpose of better defining the neuroprotective effect of said compound the inventors analyzed the neurodegenerative process with greater detail by means of analyzing neuronal death caused by apoptosis and its inhibition by said compound by means of flow cytometry in comparison with a specific inhibitor of neuronal death by apoptosis, Z-VAD-fmk, determining that compound NST0060 is capable of significantly and quantitatively inhibiting neuronal death caused by apoptosis (Figure 6).

In addition, the capacity of compound NST0060 to increase seladin-1/DHCR24 gene expression was studied since the neuroprotective effects of the increased expression of this gene with respect to Alzheimer's disease have been demonstrated (Cechi et al., J. Cell. Mol. Med. 2008; 12: 1990-2002). It has been described (Greeve et al., J. Neurosci. 2000; 20: 7345-52) that the product of the seladin-1/DHCR24 gene exerts its neuroprotective potential by means of the antiapoptotic effect by inhibition of caspase-3, and regulating cholesterol synthesis from desmosterol, which determinates the generation of a barrier against neurotoxic aggressions and prevents the production of $\beta$-amyloid. These mechanisms of action indicate that the increase of seladin-1/DHCR24 gene expression has a general neuroprotective effect, therefore those drugs that cause an increase of the expression of this gene can potentially be used in the prevention and/or treatment of neuronal death associated with neurodegenerative diseases (e.g., Alzheimer's, Parkinson's, multiple sclerosis, amyotrophic lateral sclerosis, *status epilepticus*, Huntington's, etc.) or of diseases associated with undesired oxidation or of age-associated pathological processes. The capacity of increasing seladin-1/DHCR24 gene expression by means of the administration of NST0060 is described in Example 3. It is observed in said example that compound NST0060 is capable of increasing quantitatively, significantly and in a dose-dependent manner seladin-1/DHCR24 gene expression, which is demonstrated by means of relative expression analysis using real time quantitative PCR. Said results clearly show the neuroprotective capacity of compound NST0060 (Figure 7).

For the purpose of better defining the blood-brain barrier crossing of compound NST0060, the inventors analyzed different parameters such as the theoretical lipophilicity, the percentage of crossing and the effective permeability (Figure 8, Table 1) as described in Example 4. Compound NST0060 surprisingly produced a high BBB crossing that is virtually identical to that of the positive control (verapamil) and considerably greater than the known statin with the highest BBB

crossing (simvastatin).

Furthermore, and due to the nature of compound NST0060, the inhibitory capacity of the 3-hydroxy-3-methylglutaryl coenzyme A reductase (HMGR) enzyme was studied. Surprisingly, and as shown in Example 5, the results demonstrate that compound NST0060 has an evident hypocholesterolemic effect. It can be seen in the obtained results and as shown in Figure 9 that the inhibitory activity of compound NST0060 on the HMGR enzyme is close to that of a marketed statin (simvastatin) commonly used to reduce cholesterol levels in the human population. For the purpose of better defining the hypocholesterolemic effect of the compound, the inventors analyzed the hypocholesterolemic activity with greater detail by means of analyzing cholesterol reductions in a human hepatic cell line. It can be seen in the obtained results and as shown in Figure 10 that the hypocholesterolemic activity of compound NST0060 is close to that of a marketed statin (simvastatin).

In order for a compound to be administered to the human population, its innocuousness and safety must be demonstrated. For this purpose, the inventors analyzed the biosafety of compound NST0060 in a widely used toxicological model, the zebrafish embryo, following the methodology described in the OECD C.15 protocol, as described in Example 6. In this model, the inventors compared the effect of the compound with simvastatin at concentrations greater than the doses used in clinical practice for the purpose of causing evident damage in the embryos in order to be able to better define the adverse effects of said compound. Thus, the administration of a high dose of NST0060 caused a mortality that was less than that detected with the same dose of simvastatin (Table 2). For the purpose of better defining the biosafety of the compound, the percentage of healthy larvae at the end of the experiment was studied in comparison with simvastatin, and a higher number of healthy larvae were observed in the treatments with NST0060 in comparison with simvastatin (Figure 11). For the purpose of better defining the biosafety of the compound, the percentage of heart beats according to the doses used was studied, comparing NST0060 with simvastatin, a higher reduction of the heart rate being observed in the highest evaluated dose of simvastatin than in that of NST0060 (Figure 12), indicating a higher biosafety of compound NST0037.

The anti-inflammatory activity of compound NST0060 per se and in comparison with a known statin (simvastatin) was also studied. The obtained results showed that compound NST0060 has a high anti-inflammatory potential and is surprisingly significantly better than simvastatin because it caused a significant decrease of TNF$\alpha$ and IL-1$\beta$ levels, as well as of the effects caused by injection of LPS in mice (Figures 13 to 17).

Likewise, as it is known, the administration of an excitotoxic substance induces convulsive crisis and epilepsy in the animal in some cases; for this reason, the inventors analyzed if the neuroprotective effect of compound NST0060 was accompanied by an antiepileptic and anticonvulsant effect caused by an excitotoxic substance (Figures 18 and 19), as well as of undesired oxidation caused by an excitotoxic substance (Figures 20 and 21), as well as of death caused by an excitotoxic substance (Figure 22), observing that the administration of NST0060 caused a decrease of all these parameters.

The pharmaceutical composition provided by this invention can contain compound NST0060, and/or its hydroxy acid form and/or a pharmaceutically acceptable salt of said hydroxy acid and/or a pharmaceutically acceptable prodrug or solvate of the compound or of its hydroxy acid form together with one or more pharmaceutically acceptable adjuvants, vehicles or excipients.

The term pharmaceutically acceptable "salt, prodrug or solvate" relates to any pharmaceutically acceptable salt, solvate or any other compound when administered to the recipient is capable of providing (directly or indirectly) a compound as it has been described in the present invention. Nevertheless, pharmaceutically unacceptable salts also fall within the scope of the invention, since the latter can be useful for the preparation of pharmaceutically acceptable salts. The salts and prodrugs can be prepared by means of methods known in the state of the art.

Any compound which is a prodrug of the compound of formula (I) or of its hydroxy acid form is within the scope of the invention. The term "prodrug" is used in its broadest meaning and encompasses those derivatives which are converted *in vivo* into the compounds of the invention. Such derivatives would be evident to a person having ordinary skill in the art and include the following derivatives of the present compounds: esters, amino acid esters, phosphate esters, metal sulfonate salt esters, carbamates and amides. The compounds according to the invention can be in crystalline form or as free compounds or as solvates (for example, hydrates) and it is intended that both forms are within the scope of the present invention. Solvation methods are generally known in the state of the art. In a particular embodiment, the solvate is a hydrate.

The pharmaceutical compositions containing compound NST0060, or a hydroxy acid form thereof or a pharmaceutically acceptable salt of said hydroxy acid, can be formulated in any pharmaceutical dosage form suitable for administration by the chosen administration route, e.g., oral, parenteral (subcutaneous, intramuscular, intravenous, intraperitoneal, etc.), topical, rectal route, etc. By way of a non-limiting illustration, the pharmaceutical compositions provided by this invention can be formulated in a solid pharmaceutical dosage form administered by oral route (e.g., granules, tablets, capsules, etc.), in a liquid pharmaceutical dosage form administered by oral route (e.g., solutions, suspensions, emulsions, etc.), in a pharmaceutical dosage form administered by parenteral route (e.g., solutions, suspensions, emulsions, etc.). To that end, in each case, the suitable pharmaceutically acceptable vehicles and excipients

will be chosen for the chosen pharmaceutical dosage form and route of administration, for example, binding agents, diluents, disintegrating agents, lubricants, wetting agents, etc., for the formulation of solid pharmaceutical dosage forms, and buffers, surfactants, etc., for the formulation of liquid pharmaceutical dosage forms. Said vehicles and excipients must be pharmaceutically acceptable and pharmacologically tolerable and have to be able to be combined with other components of the formulation without exerting any adverse effect on the treated subject. Information on said vehicles and excipients, as well as on said pharmaceutical dosage forms of said active ingredient, can be found in Galenic pharmacy treatises. A review of the different pharmaceutical dosage forms of drugs, in general, and of their methods of preparation can be found in the book "Treated de Farmacia Galénica" ("by C. Faulí i Trillo, 1st Edition, 1993, Luzdn 5, S.A. de Ediciones.

The pharmaceutical composition provided by this invention comprises, compound NST0060, or a hydroxy acid form thereof or a pharmaceutically acceptable salt of said hydroxy acid, in a therapeutically effective amount. In the sense used in this description, the expression "therapeutically effective amount" relates to the amount of compound calculated to cause the desired effect. The dose of compound NST0060, or a hydroxy acid form thereof or a pharmaceutically acceptable salt of said hydroxy acid, to be administered to a subject can vary within a wide range depending on a number of factors, among which the characteristics of the compound used, e.g., its biological half-life and activity, the concentration of the compound in the pharmaceutical composition, the clinical presentation of the subject, the severity of the pathology, the chosen pharmaceutical dosage form, etc., are included. The pharmaceutical composition provided by this invention can be administered one or more times a day for preventive or therapeutic purposes or, alternatively, other administration regimens can be followed, not necessarily daily but also at precise times, weekly, etc.

If desired, the pharmaceutical composition provided by this invention can be used together with other drugs, for example, drugs useful in the treatment of neurodegenerative diseases, cognitive impairment, diseases associated with undesired oxidation, age-associated pathological processes and progeria, epilepsy, epileptic seizures, convulsions, inflammation or inflammatory processes or cardiovascular diseases for the purpose of increasing the efficacy of the pharmaceutical composition provided by this invention, a combination therapy thus being generated. Said additional drugs can be part of the same pharmaceutical composition or, alternatively, can be provided as a separate pharmaceutical composition for administration at the same time (simultaneous administration) as the pharmaceutical composition provided by this invention or at different times (sequential administration) with respect to the administration of the pharmaceutical composition provided by this invention.

The following examples serve to illustrate the invention and must not be considered as limiting thereof.

EXAMPLE 1

Synthesis of 2-propylpentanoic acid (1S,3R,7S,8S,8aR)-1,2,3,7,8,8a-hexahydro-3,7-dimethyl-8-[2-[(2R,4R)-tetrahydro-4-hydroxy-6-oxo-2H-pyran-2yl]ethyl]-1-naphthalenyl ester

The compound identified as NST0060 was prepared following the methodology described in Hoffman, et al. (J. Med. Chem., 1986, 29, 849-852) for similar compounds.

1.1. Purification of lovastatin

Lovastatin was purified from an extract of natural origin by column chromatography using an hexane and ethyl acetate gradient as eluent.

1.2. Obtaining monacolin J

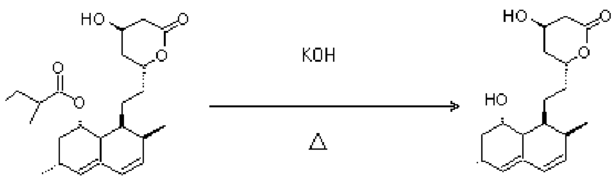


A solution of 0.7 g of potassium hydroxide in 0.5 ml of water is prepared and 3 ml of methanol are added little by little. 0.5 g of Lovastatin are subsequently added and the solution is placed under reflux for 21 hours. After the treatment of the reaction, a 50% mixture of monacolin J and the ring-opening product is obtained. NMR ($CDCl_3$) data of the monacolin J

| No. | C | H |
|---|---|---|
| **1** | 36.26 | 1.82 m |
| **2** | 30.76 | 2.37 m |
| **3** | 133.59 | 5.78 (dd, J= 9.5 and 6.1 Hz) |
| **4** | 128.50 | 5.97 (d, J= 9.5 Hz) |
| **4a** | 131.35 | |
| **5** | 130.08 | 5.52 $S_{broad}$ |
| **6** | 27.44 | 2.44 m |
| **7** | 35.84 | A: 1.87 m<br>B: 1.79 m |
| **8** | 65.24 | 4.23 (dd, J= 6.0 and 2.9 Hz) |
| **8a** | 38.70 | 2.15 m |
| **9** | 24.00 | A: 1.82 m<br>B: 1.44 m |
| **10** | 32.73 | A: 1.80 m<br>B: 1.52 m |
| **11** | 76.26 | 4.70 m |
| **12** | 36.24 | A: 1.98 m<br>B: 1.70 m |
| **13** | 62.55 | 4.34 m |
| **14** | 38.61 | A: 2.69 (dd, J= 17.6 and 4.8 Hz)<br>B: 2.59 (ddd, J= 17.6, 3.6 and 1.1 Hz) |
| **15** | 170.96 | |
| **16** | 13.98 | 0.88 (d, J= 7.0 Hz) |
| **17** | 23.74 | 1.17 (d, J= 7.5 Hz) |

1.3. Preparation of the protected derivative

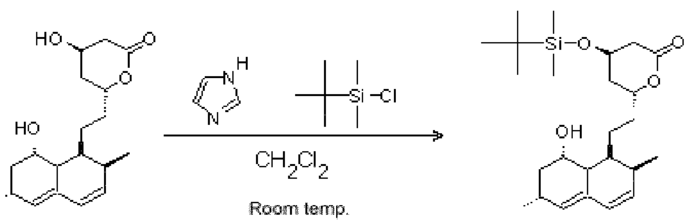


A solution of 0.5 g of monacolin J in 10 ml of dichloromethane is prepared. 0.4345 g of imidazole are added and it is stirred until dissolution. Then 0.4835 g of tert-butyl-dimethylsilane chloride dissolved in 5 ml of dichloromethane are added, and stirring is continued for 24 hours. The reaction is followed by TLC using dichloromethane-methanol (10:1) as eluent. Yield: 96%. NMR ($CDCl_3$) data of the protected derivative

| No. | C | H |
|---|---|---|
| **1** | 36.55 | 1.84 m |

(continued)

| No. | C | H |
|---|---|---|
| **2** | 30.89 | 2.41 m |
| **3** | 133.76 | 5.82 (dd, J= 9.6 and 6.2 Hz) |
| **4** | 128.48 | 6.01 (d, J= 9.6 Hz) |
| **4a** | 131.40 | |
| **5** | 130.07 | 5.58 $S_{broad}$ |
| **6** | 27.43 | 2.49 m |
| **7** | 35.87 | A: 1.95 m<br>B: 1.90 m |
| **8** | 65.34 | 4.28 (dd, J= 6.0 and 3.0 Hz) |
| **8a** | 38.91 | 2.20 m |
| **9** | 24.40 | A: 1.82 m<br>B: 1.52 m |
| **10** | 33.11 | A: 1.82 m<br>B: 1.50 m |
| **11** | 76.49 | 4.71 m |
| **12** | 36.92 | A: 1.93 m<br>B: 1.74 m |
| **13** | 63.63 | 4.33 m |
| **14** | 39.36 | A: 2.65 (dd, J= 17.4 and 4.4 Hz)<br>B: 2.59 (ddd, J= 17.4, 3.4 and 1.6 Hz) |
| **15** | 170.46 | |
| **16** | 14.01 | 0.94 (d, J= 7.0 Hz) |
| **17** | 23.88 | 1.22 (d, J= 7.5 Hz) |
| **$CH_3$**Si | -4.84 | 0.11 s |
| $CH_3$-**C** | 18.00 | |
| **$CH_3$**-C | 25.74 | 0.92 s |

1.4. Preparation of the acylated derivative

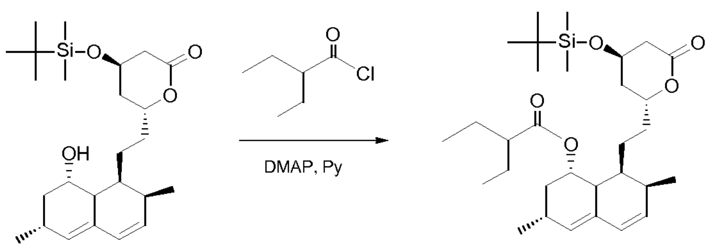


0.3 g of the protected derivative previously obtained are dissolved in a flask with inert atmosphere in 2 ml of pyridine. 0.06 g of DMAP dissolved in 2 mL of pyridine are subsequently added. The reaction flask is placed in an ice bath, and 0.378 ml of 2-ethylbutyryl chloride are added. Then it is stirred for one hour at 0°C and at room temperature for 18 hours. The reaction is followed by TLC using hexane-ethyl acetate (2:1) as eluent. Yield: 95%.

1.5. Synthesis of the final compound

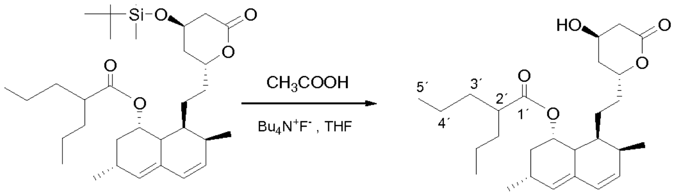


32 37

0.368 g of the derivative previously obtained are dissolved in 2 ml of THF. Then a solution of 0.16 ml of acetic acid and 2.16 ml of 1 M tetrabutylammonium fluoride is added to the reaction medium. The reaction mixture is stirred at room temperature for 16 hours. The reaction is followed by TLC using dichloromethane-acetone (6:1) as eluent. Yield: 75%. NMR ($CDCl_3$) data of the final compound

| No. | C | H |
|---|---|---|
| **1** | 36.50 | 1.60 m |
| **2** | 30.63 | 2.30 m |
| **3** | 133.07 | 5.72 (dd, J= 9.7 and 6.1 Hz) |
| **4** | 128.32 | 5.92 (d, J= 9.7 Hz) |
| **4a** | 131.53 | |
| **5** | 129.69 | 5.45 (dd, J= 3.1 and 2.9 Hz) |
| **6** | 27.41 | 2.38 m |
| **7** | 32.07 | 1.86 m |
| **8** | 67. 68 | 5.37 (dd, J= 6.2 and 3.1 Hz) |
| **8a** | 37.42 | 2.19 (dddd, J=12.0; 5.3; 2.5 and 2.5 Hz) |
| **9** | 24.63 | A: 1.46 m<br>B: 1.35 m |
| **10** | 32.96 | A: 1.80 m<br>B: 1.20 m |
| **11** | 76.54 | 4.54 m |
| **12** | 36.32 | A: 1.91 m<br>B: 1.60 m |
| **13** | 62.63 | 4.29 m |
| **14** | 38.64 | A: 2.66 (dd, J= 17.6 and 5.1 Hz)<br>B: 2.56 (ddd, J= 17.6, 3.7 and 1.5 Hz) |
| **15** | 170.47 | |
| **16** | 13.90 | 0.82 (d, J= 7.0 Hz) |
| **17** | 23.17 | 1.01 (d, J= 7.4 Hz) |
| **1’** | 176.69 | |
| **2’** | 45.67 | 2.27 m |
| **3’** | 34.64<br>34.03 | A: 1.45 m and B: 1.35 m<br>A: 1.50 m and B: 1.31 m |

(continued)

| No. | C | H |
|---|---|---|
| **4'** | 20.82<br>20.63 | A and B: 1.25 - 1.15 |
| **5'** | 14.17 | 0.81 (t, J= 7.3 Hz)<br>0.80 (t, J=7.3 Hz) |

EXAMPLE 2

Protection by NST0060 against neuronal death induced by different aggressions: oxidative stress, inhibition of protein phosphatase-1, inhibition of succinate dehydrogenase, endoplasmic reticulum stress and apoptosis

2.1. Protection by NST0060 against neuronal death induced by oxidative stress

The assay was performed on human neuroblastoma SK-N-MC cells in culture from the American Type Culture Collection (ATCC), in all cases strict rules of sterility were followed and the manipulation was performed in class II biological safety cabinets following European standard EN 12469. The cells were maintained in the following culture medium: Minimum Essential Medium Eagle (MEM) supplemented with 1 mM sodium pyruvate, 2 mM L-glutamine, 0.1 mM non-essential amino acids, 0.05 mg/ml gentamicin and 10% fetal bovine serum.

The inhibition caused by compound NST0060 with respect to cell death caused by treatment with xanthine/ xanthine oxidase which generates oxidative damage (produces free radicals such as hydrogen peroxide, superoxide anion, hydroxyl radical), which triggers cell death, was analyzed. These cells, not exceeding 15 passages, were seeded on 96-well plates treated for adherent cells with a cell concentration of $5x10^4$ cells/well; 3 wells of the plate were seeded for each assay condition.

After 24 hours of cell incubation (at 37°C and 5% $CO_2$), the cell treatments were performed with 100 μl of total volume for the following conditions:

- Control: culture medium (medium)
- Xanthine/xanthine oxidase (XXO): medium plus 10 μM xanthine / 60 mU/mL xanthine oxidase, causing death of 50% of the cells.
- XXO plus NST0060: medium plus XXO (10 μM / 60 mU/mL) plus NST0060 at 1, 4, 10, 20, 40 or 100 μM.

The cells were incubated (at 37°C and 5% $CO_2$) with these treatments for 22 hours, after which time WST-1 reagent (Roche) was added. The WST-1 test is based on the measurement of metabolic activity. Cell damage causes the loss of the ability of cells to obtain the energy necessary to maintain their metabolic functions and cell growth, therefore the metabolically active (live) cells reduce tetrazolium salt to formazan by means of the succinate-tetrazolium reductase system (of the mitochondrial respiratory chain). The formazan which is formed can be detected colorimetrically since it has an absorbance of 440 nm. The reading was taken in a plate reader at 440 nm 2 hours after adding the reagent.

The obtained results are shown, as it can be seen in Figure 1, as the percentage of cell death for each treatment relating to death caused by XXO. Protection against death was observed with concentrations from 1 to 40 μM of NST0060, the differences with respect to XXO being statistically significant according to the Student's t-test, and reaching maximum protection of 80% at 10 μM. These results indicate that compound NST0060 shows a protective effect against the death of human cells of neuronal origin caused by oxidative stress.

2.2. Protection by NST0060 against neuronal death induced by inhibition of protein phosphatase-1 (PP1)

The assay was performed on human neuroblastoma SK-N-MC cells in culture, maintained as described in section 2.1.

The inhibition caused by compound NST0060 with respect to cell death caused by treatment with okadaic acid (OA) which inhibits the activity of protein phosphatase-1 (PP1) was analyzed. OA is one of the most widely used drugs for studying the phosphorylation mechanism of the tau protein, involved in the pathogenesis and progression of AD. Inhibition of PP1 causes cytoskeleton alterations and mitochondrial damage. These cells, not exceeding 15 passages, were seeded on 96-well plates treated for adherent cells with a cell concentration of $5x10^4$ cells/well; 3 wells of the plate were seeded for each assay condition.

After 24 hours of cell incubation (at 37°C and 5% $CO_2$), the cell treatments were performed with 100 μl of total

volume for the following conditions:

- Control: culture medium (medium)
- Okadaic acid (OA): medium plus 20 nM OA, causing death of 50% of the cells.
- OA plus NST0060: medium plus OA (20 nM) plus NST0060 at 1, 4, 10, 40 or 100 μM.

The cells were incubated (at 37°C and 5% $CO_2$) with these treatments for 22 hours, after which time WST-1 reagent (Roche) was added. The WST-1 test is based on the measurement of metabolic activity (method described in section 2.1).

The obtained results are shown, as it can be seen in Figure 2, as the percentage of cell death for each treatment relating to death caused by OA. Statistically significant protection against death was observed with 4 and 10 μM of NST0060, reaching 83% at 10 μM. These results indicate that compound NST0060 shows a protective effect against the death of human cells of neuronal origin caused by inhibition of PP1.

2.3. Protection by NST0060 against neuronal death induced by inhibition of succinate dehydrogenase

The assay was performed on human neuroblastoma SK-N-MC cells in culture, maintained as described in section 2.1.

The inhibition caused by compound NST0060 with respect to cell death caused by treatment with 3-nitropropionic (3-NP) acid was analyzed. 3-NP is an irreversible inhibitor of the succinate dehydrogenase enzyme which in animal models causes oxidative stress and apoptotic cell death in the striatum and mimics neurochemical and anatomical changes associated with Huntington's disease (HD). These cells, not exceeding 15 passages, were seeded on 96-well plates treated for adherent cells with a cell concentration of $5x10^4$ cells/well; 3 wells of the plate were seeded for each assay condition.

After 24 hours of cell incubation (at 37°C and 5% $CO_2$), the cell treatments were performed with 100 μl of total volume for the following conditions:

- Control: culture medium (medium)
- 3-Nitropropionic (3-NP): medium plus 30 μM 3-NP, causing death of 50% of the cells.
- 3-NP plus NST0060: medium plus 3-NP (30 μM) plus NST0060 at 1, 4, 10, 40 or 100 μM.

The cells were incubated (at 37°C and 5% $CO_2$) with these treatments for 22 hours, after which time WST-1 reagent (Roche) was added. The WST-1 test is based on the measurement of metabolic activity (method described in section 2.1).

The obtained results are shown, as it can be seen in Figure 3, as the percentage of cell death for each treatment relating to death caused by 3-NP. Statistically significant protection against death was observed in the range of 4 to 40 μM of NST0060, reaching 32% at 40 μM. These results indicate that compound NST0060 shows a protective effect against the death of human cells of neuronal origin by inhibition of the succinate dehydrogenase enzyme.

2.4. Protection by NST0060 against neuronal death induced by endoplasmic reticulum stress

The assay was performed on human neuroblastoma SK-N-MC cells in culture, maintained as described in section 2.1.

The inhibition caused by compound NST0060 with respect to cell death caused by treatment with tunicamycin generating endoplasmic reticulum stress was analyzed. Tunicamycin is an inhibitor of protein N-glycosylation, causing abnormal protein folding in the endoplasmic reticulum, therefore said proteins are accumulated and cause stress, resulting in cell death. These cells, not exceeding 15 passages, were seeded on 96-well plates treated for adherent cells with a cell concentration of $5x10^4$ cells/well; 3 wells of the plate were seeded for each assay condition.

After 24 hours of cell incubation (at 37°C and 5% $CO_2$), the cell treatments were performed with 100 μl of total volume for the following conditions:

- Control: culture medium (medium)
- Tunicamycin (TM): medium plus 24 μM tunicamycin, causing death of 50% of the cells.
- TM plus NST0060: medium plus TM (24 μM) plus NST0060 at 1, 4, 10, 40 or 100 μM.

The cells were incubated (at 37°C and 5% $CO_2$) with these treatments for 22 hours, after which time WST-1 reagent (Roche) was added. The WST-1 test is based on the measurement of metabolic activity (method described in section 2.1).

The obtained results are shown, as it can be seen in Figure 4, as the percentage of cell death for each treatment relating to death caused by TM. Statistically significant protection against death was observed with from 1 to 40 μM of NST0060, reaching 42% at 4 μM. These results indicate that compound NST0060 shows a protective effect against the death of human cells of neuronal origin caused by endoplasmic reticulum stress.

<u>2.5. Treatment with NST0060 inhibits the activation of caspase 3/7 in a cell model of apoptosis</u>

The assay was performed on human neuroblastoma SK-N-MC cells in culture, maintained as described in section 2.1.

The induction of apoptosis on the cells in culture was performed with camptothecin (CPT), which triggers the activation of cell apoptosis. Apoptosis was quantified by means of the measurement of the activation of effector caspases, caspase 3 or caspase 7, for which a kit for the fluorometric detection of active caspase 3/7 (Apo-ONE® Homogeneous Caspase-3/7, Promega) was used. Active caspases 3 or 7 of the cells cause the rupture of a substrate, which leads to fluorescence emission, which is read by means of a fluorometer. The effect of the pretreatment of compound NST0060 at 10 and 40 μM on the activation of caspase 3/7 produced by 50 μM of camptothecin (CPT) in the SK-N-MC cells was thus analyzed. These cells, not exceeding 15 passages, were seeded on 96-well plates treated for adherent cells with a cell concentration of $5x10^4$ cells/well; 3 wells of the plate were seeded for each assay condition.

After 24 hours of cell incubation (at 37°C and 5% $CO_2$), the pretreatment with NST0060 at 10 and 40 μM was performed.

After 24 hours, the cell treatments were performed with 100 μl of total volume for the following conditions:

- Control: culture medium (medium)
- Camptothecin (CPT): medium plus 50 μM CPT to cause apoptosis, both in points of the assay pretreated with NST0060 at 10 or 40 μM and in cells that are not pretreated
- CPT plus Z-VAD-fmk: medium plus CPT (at the same concentration as in the preceding condition) plus Z-VAD-fmk at 50 μM, as positive control of the inhibition of the activation of caspases.

The cells were incubated (at 37°C and 5% $CO_2$) with these treatments for 6 hours, after which cell lysis buffer and the caspase substrate at the concentrations specified by the manufacturer were added; it was incubated at room temperature for 30 minutes and then frozen at -20°C overnight. Fluorescence was measured the next day (499/521 nm, Exci/Emi).

Figure 5 shows the percentage of activation of caspase 3/7 with respect to the control cells of the different treatments. An inhibition of caspase 3/7 with respect to camptothecin of 58 and 32% at 10 and 40 μM of NST0060, respectively, was observed, which demonstrates a functional anti-apoptotic effect of this compound. Z-VAD-fmk completely inhibited the activation of caspase 3/7 as a caspase inhibitor.

<u>2.6. Treatment with NST0060 inhibits DNA fragmentation in a cell model of apoptosis</u>

Due to the results obtained and presented in the preceding sections of this example, the inventors decided to analyze the effect of NST0060 in a new cell model of apoptosis by measuring DNA fragmentation.

The assay was performed on human neuroblastoma SK-N-MC cells in culture, maintained as described in section 2.1.

The inhibition caused by compound NST0060 with respect to apoptosis caused by treatment with camptothecin (CPT), which inhibits the topoisomerase I enzyme, prevents DNA duplication and triggers apoptotic cell death. These cells, not exceeding 15 passages, were seeded on 6-well plates treated for adherent cells with a cell concentration of $7x10^5$ cells/well; 2 wells of the plate were seeded for each assay condition.

After 24 hours of cell incubation (at 37°C and 5% $CO_2$), cell pretreatment was performed with 10 and 40 μM of NST0060 for 24 hours; they were subsequently treated with 50 μM CPT for 6 hours. Furthermore, Z-VAD-fmk at 50 μM was used as positive control for inhibition.

After treatment, the cells were collected along with their culture medium and centrifuged at 300xg for 5 minutes. The medium was removed, a washing was performed with PBS and the cells were fixed for 2 minutes with 500 μl of 70% ethanol at - 20°C. Once fixed, they were centrifuged at 400xg for 5 minutes, washed with PBS and 0.05 mg/ml of propidium iodide, diluted in cycling buffer (0.1% sodium citrate, 0.3% Nonidet P-40 and 0.02 mg/ml RNAse) were added and they were incubated for 1 hour at 37°C. After this time they were analyzed by flow cytometry, comparing the fluorescence of propidium iodide with respect to the amount of DNA. The percentage of apoptosis was measured on the sub-G1 region of each of the conditions.

The obtained results are shown, as it can be seen in Figure 6, as the percentage of apoptosis (by DNA fragmentation) of each treatment relating to apoptosis caused by CPT. Maximum protection of 41% was observed at 10 μM

of NST0060. In turn, Z-VAD-fmk specifically inhibited apoptosis caused by CPT. These results indicated that compound NST0060 shows a protective effect against apoptosis in human cells of neuronal origin.

EXAMPLE 3

Induction of the cell expression of the 24-dehydrocholesterol reductase gene (Seladin-1/DHCR24) due to treatment with NST0060

The assay was performed on human neuroblastoma SK-N-MC cells in culture from the American Type Culture Collection (ATCC), in all cases strict rules of sterility were followed and the manipulation was performed in class II biological safety cabinets following European standard EN 12469. The cells were maintained in the following culture medium: Minimum Essential Medium Eagle (MEM) supplemented with 1 mM sodium pyruvate, 2 mM L-glutamine, 0.1 mM non-essential amino acids, 0.05 mg/ml gentamicin and 10% fetal bovine serum.

Real-time quantitative RT-PCR was used to analyze the expression of the seladin-1/DHCR24 gene. The SK-N-MC cells were treated for 24 hours in the absence (control) or presence of NST0060 at 1, 4, 10 or 40 μM. The total RNA was extracted by means of the High Pure RNA Isolation kit (Roche) and the RNA amount and quality were analyzed by means of spectrophotometry in the Infinite 200 NanoQuant system (Tecan). The RT-PCR was performed by means of two steps, first, the mRNA was transformed to cDNA using the RNA to cDNA kit (Applied Biosystem) and the gene expression was subsequently analyzed by means of TaqMan probes using the validated probes Hs00207388_m1 for seladin-1/DHCR24 and Hs99999905_m1 for GAPDH (used to normalize the results), both of which are human-specific, in the 7500 Fast Real-Time PCR System equipment (Applied Biosystem). An assay was performed in triplicate. The relative amount of gene expression was determined by using the ΔΔCt method with the SDS v2.1.1 software (Applied Biosystem); the expression of GAPDH was used to normalize results.

The obtained results are shown in Figure 7, where the increase of seladin-1/DHCR24 gene expression with treatment with NST0060 at all the tested concentrations is observed, causing a 2.5-fold increase with respect to the control at 4 μM NST0060. These results indicate that the treatment with NST0060 causes the increase of seladin-1/DHCR24 gene expression, which is related to cholesterol biosynthesis and to anti-apoptotic capacity.

EXAMPLE 4

Blood-brain barrier crossing of NST0060 compared to simvastatin in an *in vitro* assay

The purpose of the assay was to predict if compound NST0060 is capable of crossing the blood-brain barrier (BBB). To that end, the BBB was mimicked in an *in vitro* system that allowed evaluating the compound without using cells, and where the so-called PAMPA (Parallel Artificial Membrane Permeation Assay) was used, using a sandwich system considering the crossing of compounds by means of passive diffusion. Verapamil, a compound with high permeability, was used as positive control, whereas theophylline, a compound which does not cross the BBB, was used as negative control. Simvastatin, a compound with a similar structure that was previously known to be capable of crossing the BBB to a certain extent, was tested together with NST0060.

A mixture of marketed lipids derived from pig brain with a phospholipid composition very similar to that which forms the barrier and known as PBL (Porcine Polar Brain Lipid) was used to mimic the blood-brain barrier. This compound was stored at -20°C dissolved in dodecane at 100 μg/ml in glass vials. Verapamil and theophylline were prepared at 10 mM in DMSO. The acid forms of NST0060 and simvastatin were prepared in water and activated with 0.1 N NaOH at 4°C for 12 hours.

At the time of the assay, 1.5 ml of the compounds to be evaluated and of the controls were prepared, in all cases at 100 μM from the stocks in a phosphate buffer at pH 7.4 which contained monobasic sodium phosphate (0.41 M) and dibasic potassium phosphate (0.287 M). To that end, a 1/100 dilution of the compounds was performed, being the DMSO content in all cases 1%.

Five microliters of PBL at 20 μg/ml prepared from the frozen stock at 100 μg/mL were added in a 96-well filter plate, with a PVDF membrane and a pore size of 45 μm (MAIPN4550, Millipore). After 2 minutes, 300 μl of the phosphate buffer used to dilute the compounds were added. This plate was considered the acceptor plate and was placed in the top part of the sandwich. 300 μl of the compounds were added at 100 μM and in triplicate on another 96-well plate which is assembled with the previous one (MATRNP550, Millipore). Furthermore, a blank which included, only with 1% DMSO in the phosphate buffer used. This plate was called the donor plate. The acceptor plate was placed on the donor plate forming the sandwich system. The compounds object of study diffused from the wells of the donor plate to the corresponding wells of the acceptor plate for 18 hours during which the system remained intact. The remaining compound prepared was stored in the same humidity, temperature and darkness conditions as the sandwich system formed by the plates. After that time, 100 μl from the wells of the donor plates and acceptor plates were transferred to a special 96-well plate for UV reading. Furthermore, 100 μl of the compounds prepared for performing the assay and stored in the same manner were transferred in triplicate to the plates (basal wells). The UV plate was introduced in a spectrophotometer in which a scan was carried out in UV from 230 to 498 nm, with readings every 4 nm. The percentage of barrier crossing as well as the effective permeability ($P_e$) were calculated from the spectrophotometric data. The following formula was applied to calculate the $P_e$:

$$Pe = C\,x - Ln\left(1 - \frac{[Drug]_{acceptor}}{[Drug]_{equilibrium}}\right)$$

where:

$$C = \frac{V_D \times V_A}{(V_D \times V_A) \times Area \times Time}$$

$[Drug]_{acceptor}$ = Absorbance of the acceptor well
$[Drug]_{equilibrium}$ = Absorbance of the mean of the basal wells / 2
$V_A$= Volume of the acceptor well = 0.3 $cm^3$
$V_D$= Volume of the donor well = 0.3 $cm^3$
Area = 0.24 $_{cm}{}^2$
Time = 64,000 s

The theoretical calculation of the lipophilicity of a compound can be obtained by calculating the logarithm of the octanol/water partition coefficient, abbreviated as cLogP, which was calculated by means of the CLOGP program (OSIRIS Property Explorer) by entering the chemical structures in the software.

Figure 8 shows the obtained results, where it can be seen that compound NST0060 in its hydroxy acid form has a degree of BBB penetration that is very close to the positive control (verapamil) and is furthermore greater than that of simvastatin. This data correlates to the higher lipophilicity of compound NST0060. The following table (Table 1) shows the parameters obtained in this study (mean±SD), and it reflects the barrier crossing (% BBB crossing), effective permeability ($P_e$), the number of experiments conducted (n) and the calculation of the theoretical lipophilicity (calculated theoretically by means of the CLOGP, OSIRIS Propert Explorer) (cLogP).

| | % BBB crossing | $P_e$(cm/s) | n | cLogP |
|---|---|---|---|---|
| **Verapamil** | 32.0±4.6 | 10.1±2.4 | 6 | 5.32 |
| **Theophylline** | 1.6±0.7 | 0.3±0.1 | 6 | -0.09 |
| **Simvastatin** | 17.8±0.6 | 4.3±0.3 | 6 | 4.61 |
| **NST0060** | 29.8±2.4 | 8.8±1.2 | 6 | 5.48 |

Surprisingly, the results shown in this example clearly show a highly efficient BBB crossing of compound NST0060.

EXAMPLE 5

Hypocholesterolemic effect of compound NST0060 5.1. Inhibition of 3-hydroxy-3-methylglutaryl coenzyme A reductase (HMGR) enzyme activity *in vitro* by NST0060 compared to simvastatin

The purpose of the assay was to determine the degree of inhibition of HMGR, a key enzyme in cellular cholesterol synthesis and in the physiological regulation of said synthesis, by compound NST0060. The effect of this compound was compared with that of a statin, simvastatin, for which a potent inhibitory effect on HMGR has already been described.

During the reaction, HMGR uses NADPH as a reducing agent and 3-hydroxy-3-methylglutaryl coenzyme A (HMGCoA) as a substrate. Mevalonic acid, which is used as a substrate for the following reaction to continue cholesterol

synthesis, is obtained as a reaction product. The enzymatic reaction under study is schematized below:

$$HMGCoA + 2NADPH + 2H^+ \rightarrow \text{Mevalonic acid} + 2NADP^+ + CoASH$$

This *in vitro* assay is based on the spectrophotometric measurement of the decrease in absorbance at 340 nm, depicting oxidation of NADPH by the catalytic subunit of HMGR in the presence of the HMGCoA reductase substrate.

The compounds under study were prepared in ultrapure water at 10 mM and were activated with 0.1 N NaOH for 12 hours at 4°C. The assay was carried out in a 96-well plate, with a total reaction volume of 200 μl. The reaction buffer (50 mM $KH_2PO_4$, 1 M KC1, 2 mg/ml Bovine Serum Albumin [BSA] and 5 mM DTT, at pH=7.3) was prepared at the time the reaction was carried out and was maintained at 37°C. The following were included in the assays:

- <u>A blank</u>: Lacking HMGR which reports on the stability of NADPH during the reaction.
- <u>A control</u>: Containing all the components of the reaction but lacking the test compound.
- <u>Test compounds</u>: NST0060 or simvastatin dissolved in the reaction buffer at various concentrations.

The effect of both compounds was evaluated at 0.0004, 0.001, 0.004, 0.01, 0.04, 0.1, 0.4, 1, 4, 10 and 40 μM in five assays in duplicate and in parallel for both compounds. The test compounds were added to the reaction buffer in the plate, and the DMSO concentration was standardized at 10 μl, having demonstrated previously the non-interference thereof during the reaction. The concentrations of the reagents involved in the reaction mixture were the following and were added dissolved in reaction buffer in the indicated order:

- <u>HMGCoA</u>: 0.2 mM
- <u>HMGR</u> (catalytic domain bound to GST proteins): 3 μU/reaction
- <u>NADPH</u>: 0.2 mM

NADPH is the reaction-triggering compound, so it was added simultaneously in all the wells. A blank and a control were included each time NADPH was added. After brief stirring, the plate was read in a spectrophotometer at 340 nm, 37°C and with a kinetic program that allowed reading every 20 seconds for 1200 seconds.

The inhibitory capacity of the different compounds on HMGR enzyme activity was calculated by means of determining the 50% inhibition constants ($IC_{50}$), based on the curves shown in Figure 9, because the decrease in absorbance at 340 nm allows calculating the percentage of HMGR enzyme activity with respect to the control. The $IC_{50}$ was determined by means of the method Trimmed Spearman-Karber (Version 1.5). The results confirm the inhibitory effect of simvastatin on HMGR ($IC_{50}$: 83.3±24.3 nM, five independent assays in duplicate), whereas NST0060 surprisingly showed high inhibition power on the enzyme ($IC_{50}$: 126.0±34.6 nM, five independent assays in duplicate).

<u>5.2. Effect of NST0060 and simvastatin in their acid forms on intracellular total cholesterol levels in the human hepatic cell line</u>

Based on the results of the preceding section, the inventors decided to investigate if the inhibitory activity of the HMGR enzyme on the compound NST0060 corresponded with a variation of total cholesterol in human hepatic lines.

To that end an assay was conducted which had the purpose of determining if compound NST0060 in its active form was capable of modifying total cholesterol levels in the human hepatocarcinoma line HepG2. The effect of NST0060 was compared to that of a known statin, simvastatin. To carry out this assay, the compounds were prepared at 10 mM in ultrapure water and were activated by means of 0.1 N NaOH for 12 hours at 4°C. The compounds were stored at -20°C until they were used.

The HepG2 line was obtained from the American Type Culture Collection (ATCC). The cells maintained in passage were seeded in 96-well plates ($4x10^4$ cells/well) in MEM supplemented with 10% FBS, 2 mM L-glutamine, 1 mM sodium pyruvate, 0.1 mM non-essential amino acids and 0.05 mg/mL gentamicin. After 24 hours of the seeding, the cells were deprived of FBS for 8 hours. The cells were then incubated with the treatments at various concentrations for 20 hours. After the incubation period, the cells were washed with PBS and lysed with a phosphate buffer with 0.5% Triton X-100. The lysis was completed by means of a double freezing-thawing.

The total cholesterol was quantified by means of enzymatic and fluorometric techniques. 25 μL of each lysate were transferred to a 96-well plate together with a standard cholesterol curve (from 0.08 μg/mL to 10 μg/mL). 75 μL of the reactive mixture prepared based on 0.05 M MES (pH 6.5) containing cholesterol oxidase (0.5 U/mL), cholesterol esterase (0.8 U/mL), horseradish peroxidase (4 U/mL) and ampliflu red (20 μg/mL) were added to the samples and incubation was performed for 15 minutes at 37°C. The fluorescence intensity was determined at 530 nm of excitation and 580 nm of emission by means of a fluorometer. The results were standardized by amount of protein, which was determined by means of the BCA technique.

Figure 10 shows how NST0060 surprisingly caused reductions in total cholesterol in the cells similar to those exerted by simvastatin. This data confirms the hypocholesterolemic capacity of NST0060 as a consequence of the inhibition exerted on HMGR and shown in the preceding section.

EXAMPLE 6

Study of the biosafety of compound NST0060 in zebrafish embryo 6.1. Analysis of the survival rate in zebrafish embryos after of NST0060 treatment in comparison with simvastatin treatment. Acute toxicity assay

To evaluate the biosafety of compound NST0060, its toxicological effects on the zebrafish embryo model were analyzed by means of determining the safety parameters according to the OECD C.15 protocol "Fish, short-term toxicity test on embryo and sac -fry stages", semi-static method.

The fertilized eggs were obtained by natural mating of zebrafish (*Danio rerio*, AB strain). A total of 8-10 pairs were used for each cross and a total of 200-250 eggs were generated on average per pair. The eggs were collected immediately after spawning and washed with dilution water (0.29 g/L $CaCl_2$•$2H_2O_2$, 0.12 g/L $MgSO_4$•$7H_2O_2$, 0.065 g/L $NaHCO_3$, 0.006 g/L KCl), the pH being adjusted to 7.8 ± 0.2, in accordance with EC Regulation 440/2008, OECD method C.1, and deposited in a Petri dish.

To assure exposure in the earliest stages of development, the eggs were quickly transferred (a minimum of 50 per condition) to another Petri dish with solutions of the compounds to be tested. Subsequently, only the fertilized eggs (30 per experimental condition) were transferred from the Petri dishes to the exposure chambers (M96 microtiter plates) by means of pipettes and exposed to the substances to be evaluated (NST0060 and simvastatin), with dilution water (controls) or with different concentrations of the treatments (from 1 to 100 mg/Kg). The embryos were incubated without additional aeration, at the suitable temperature (25±1°C). Furthermore, strict precautions were taken to prevent contamination during manipulation processes. The studies were carried out at 72 hours post-treatment, where the treatment was renewed every day, thereby performing a semi-static assay. Each experiment was performed with 10 replicas of 3 embryos each according to the following treatment regimens:

- ○ 30 control animals treated with dilution water and with daily replacement until the end of the assay.
- ○ 30 animals treated with compound NST0060 (3 embryos for every ten replicates) diluted in dilution water at doses between 1 and 100 mg/Kg and with daily replacement of the substance during the days that the assay lasted.
- ○ 30 animals treated with the compound simvastatin (3 embryos for every ten replicates) diluted in dilution water at doses between 1 and 100 mg/Kg and with daily replacement of the substance during the days that the assay lasted.

After the time of exposure to the substances under study, the mortality record of the larvae was determined, results being shown in Table 2. Said table shows the number of larvae used for the experiment, the dose in mg/Kg (by weight of the animal), treatment time and the number of dead larvae with respect to the total.

The results of the study showed that both compounds have a very similar toxicological profile concerning the parameter of inducing mortality in zebrafish larvae (72 hpf), observing 7 deaths out of 30 animals in the NST0060 group at the dose of 10 mg/Kg, 16 being observed at the same dose and time with simvastatin. No statistically significant differences between both treatment groups were detected (Student's t-test).

| Group (NST0060) | Embryos | Dose (mg/kg) | Time (Days) | Mortality |
|---|---|---|---|---|
| 1 | 30 | Vehicle[a] | 3 | 0/30 |
| 2 | 30 | 1 | 3 | 0/30 |
| 3 | 30 | 3 | 3 | 0/30 |
| 4 | 30 | 10 | 3 | 7/30 |
| 5 | 30 | 30 | 3 | 30/30 |
| 6 | 30 | 100 | 3 | 30/30 |
| Group (simvastatin) | Embryos | Dose (mg/kg) | Time (Days) | Mortality |
| 1 | 30 | Vehicle[a] | 3 | 0/30 |
| 2 | 30 | 1 | 3 | 0/30 |
| 3 | 30 | 3 | 3 | 0/30 |
| 4 | 30 | 10 | 3 | 16/30 |
| 5 | 30 | 30 | 3 | 30/30 |
| 6 | 30 | 100 | 3 | 30/30 |

[a] Embryo water (dilution water).

The results shown in the preceding table show that compound NST0060 is surprisingly at least as safe as simvastatin.

6.2. Analysis of the percentage of healthy larvae after exposure to compound NST0060 in comparison with simvastatin. Acute toxicity assay

Based on the results of the preceding section, the inventors decided to investigate if the variations in mortality resulting from the treatments were corroborated with the determination of the percentage of healthy larvae, defined as the number of living larvae free of symptoms of external physiopathological anomalies (morphological and/or behavioral), the latter being a parameter determining the biosafety of a substance under study and it is complementary to survival rates.

As shown in Figure 11, differences were observed in the percentages of healthy larvae between the two treatments evaluated at doses of 1 and 3 mg/Kg, being 100±0.0% for compound NST0060 and 50.0±22.4% for simvastatin at the dose of 1 mg/Kg and reaching 86.7±6.2% for NST0060 and 50.0±22.4% for simvastatin at the dose of 3 mg/Kg, which surprisingly indicates better biosafety of compound NST0060 in the model of zebrafish larva.

These results indicate that NST0060 has a better biosafety profile than simvastatin.

6.3. Analysis of the variation in cardiotoxicity of compound NST0060 in comparison with simvastatin according to dose

Based on the results of the preceding sections, the inventors decided to investigate if the higher biosafety of compound NST0060 in comparison with simvastatin was corroborated by means of analyzing cardiotoxicity after treatment with the different compounds and at several doses. The study of this parameter (cardiotoxicity) not only determines the heart rate of the animals but also it allows determining heart beat alterations (tachycardia, bradycardia, etc.) or heart development anomalies (pericarditis, atrophy, hypertrophy, etc.).

The determination of the heart rate of the zebrafish embryos or larvae is performed visually. The heart beat (with the help of a manual counter) is counted for a period of 15 seconds by means of a magnifying glass (magnifications of 4-40x). The heartbeat of 12 of the 30 animals used per treatment in total is counted. The number of beats obtained is multiplied by 4 to obtain the number of heart beats per minute of the animal.

The results are shown in Figure 12, where it can be seen that the percentage of the heart rate of embryos or larvae with respect to the control depends on the dose used. At 72 hpt, a significant decrease of the heart rate in larvae

treated with simvastatin with respect to the controls and at doses of 3 and 10 mg/Kg is observed. In relation to NST0060, the decrease of the heart rate is detected exclusively at the dose of 10 mg/Kg, but surprisingly not at the dose of 3 mg/Kg. In addition, the comparison of the heart rate at the doses of 1 and 3 mg/Kg for NST0060 and simvastatin surprisingly shows that NST0060 is more biosafe than simvastatin. Therefore and in general, NST0060 performed better than simvastatin in the cardiotoxicity studies.

EXAMPLE 7

Anti-inflammatory effect of NST0060 in a model of systemic inflammation in mice

7.1. Comparative study of the anti-inflammatory effect of NST0060 and simvastatin.

The investigators considered necessary to evaluate alternative activities with respect to hypocholesterolemic and neuroprotective activities given the characteristics and properties of the molecule. For this reason the anti-inflammatory capacity of compound NST0060 in comparison with simvastatin, a compound for which an anti-inflammatory effect has been described, was evaluated. To evaluate this activity, a model of systemic shock in mice induced by lipopolysaccharide (LPS), a component of the gram negative bacteria structure that induces a cytokine-mediated, mainly TNF$\alpha$-mediated, inflammatory response was used.

All the animals included during the experimental process were 12-week old male C57BL6 mice. The experiments were carried out strictly complying with the standard entitled Guidance on the Operation of Animals (Scientific Procedures, Act. 1986*).* The animals were subjected to their respective quarantine period and were treated with maximum precaution to minimize possible contaminations during treatments and handling.

The mice were split up into three groups: control group (n =8); simvastatin group (n = 8); NST0060 group (n = 8). The mice were i.p. administered two doses of 50 mg/kg of simvastatin or of NST0060 (in their lactone forms) spaced out 12 hours from one another. In both cases 0.5% methyl cellulose in physiological saline, which was administered to mice in the control group with the same administration regimen, was used as a vehicle. Peripheral systemic inflammation was induced one hour after the last treatment by means of a single i.p. injection of LPS at 8 mg/kg. Two hours later blood was taken and plasma was obtained, which was used to evaluate the anti-inflammatory effect by analyzing plasma concentrations of TNF$\alpha$ by means of ELISA.

Figure 13 shows that at 2 hours post-inoculation of LPS there is a dramatic increase in TNF$\alpha$ blood levels. In turn, the administration of both simvastatin and NST0060 causes a large decrease in said levels that is significant with respect to the control group. Compound NST0060 was surprisingly stronger at this level, leading to statistically significant TNF$\alpha$ plasma level reductions with respect to the group of simvastatin. Therefore and in general, NST0060 showed a better anti-inflammatory performance than simvastatin.

7.2. Effect of NST0060 on inflammatory markers, survival and inflammatory response by symptomatological evaluation in a model of systemic shock in mice

Having demonstrated the surprising anti-inflammatory capacity of compound NST0060, the investigators considered convenient to further study the said anti-inflammatory effect and to determine in the same model of systemic shock the effect of compound NST0060 on survival and symptomatology of the treatments.

To that end an additional assay was carried out in which all the animals included were 12-week old male C57BL6 mice. The experiments were carried out strictly complying with the standard entitled Guidance on the Operation of Animals (Scientific Procedures, Act. 1986*).* The animals were subjected to their respective quarantine period and were treated with maximum precaution to minimize possible contaminations during treatments and handling.

The mice were split up into three experimental groups: control group (n = 8); control+LPS group (n = 16); NST0060+LPS group (n = 16). The mice were i.p. administered two doses of 50 mg/kg of NST0060 (in its lactone form) spaced out 12 hours from one another. 0.5% methyl cellulose in physiological saline, which was administered to the mice in the control groups with the same administration regimen, was used as a vehicle. Peripheral systemic inflammation was induced one hour after the last treatment by means of a single i.p. injection of LPS at 8 mg/kg. Half the mice in which systemic shock was induced (8 animals from the control+LPS group and 8 animals from the NST0060+LPS group) were sacrificed 2 hours after the inoculation of the LPS. Blood was taken and plasma was obtained, and both were frozen. Blood was taken from the other half of the animals treated with LPS and plasma was obtained 4 hours after the injection of LPS. These animals were observed for 15 days thereafter to determine mortality and evaluate damage by means of a score ranging from 0 to 3 (0 = no symptomatology; 1 = mild lethargy and hypothermia; 2 = severe lethargy, diarrhea and tremors; 3 = absolute immobility). The control animals given an i.p. injection of physiological saline instead of LPS were sacrificed at the end of the study after taking blood at 2 hpi and 4 hpi for the subsequent comparison of results. The concentration of the inflammatory cytokines TNF$\alpha$ and IL-1$\beta$ was evaluated in the plasma obtained by

means of ELISA.

Figure 14 shows the survival rate of the animals corresponding to the different treatment groups, showing that the mortality induced by LPS is prevented in a statistically significant manner by the administration of NST0060, which indicates its anti-inflammatory activity. This protective effect is also reflected in a lower severity of the systemic shock process, as it can be seen in Figure 15 showing the score associated with the symptomatology exhibited by the surviving animals after the injection of LPS, being statistically significant at 24 hours. Additionally, the treatment with NST0060 causes a decrease in the inflammatory response at both 2 and 4 hours post-LPS, as it can be seen in Figure 16. Treatment with NST0060 further causes a statistically significant decrease in cytokine IL-1$\beta$ plasma levels at 2 hours post-LPS, as it is shown in Figure 17.

Therefore and in general, compound NST0060 has high anti-inflammatory activity which is surprisingly higher than that of simvastatin.

EXAMPLE 8

<u>Antiepileptic effect of NST0060 against the action of an excitotoxic substance</u>

The administration of an excitotoxic substance in animals is known to sometimes induce epileptic seizures and convulsions in the animals. The inventors therefore decided to investigate if the neuroprotective effect of NST0060 was accompanied by an antiepileptic effect caused by an excitotoxic substance.

All the animals included during the experimental process were 12-week old males from the FVB/NHan strain. The experiments were carried out strictly complying with the standard entitled Guidance on the Operation of Animals (Scientific Procedures, Act. 1986*).* The animals were subjected to their respective quarantine period and were treated with maximum precaution to minimize possible contaminations during inoculations and handling.

The animals were intraperitoneally inoculated with 25 mg/kg of kainate (KA) dissolved in PBS. Fourteen animals were pretreated at 24 and 0.5 hours before inoculation with KA by means of intraperitoneal injection with 0.25% methyl cellulose in PBS (vehicle+KA administration regimen), and 14 were pretreated at 24 and 0.5 hours before inoculation with KA by means of intraperitoneal injection with compound NST0060 at a dose of 50 mg/kg (NST0060+KA administration regimen). After inoculation, the animals were individually housed in trays to monitor them. The maximum level of epilepsy of the animals was recorded during the observation according to the Racine scale every ten minutes and for at least 120 minutes post-inoculation (m.p.i.).

Comparative studies of the epileptogenic phenomena between treatment with the vehicle (Vehicle+KA) and with NST0060 (NST0060+KA) were subsequently conducted. As shown in the following table (Table 3), differences were observed in the percentage of animals that entered in *status epilepticus,* i.e., they presented tonic-clonic seizures, within the NST0060+KA group, which was 36% (5 out of 14) with respect to the vehicle+KA group, which was 93% (13 out of 14), demonstrating that compound NST0060 is anticonvulsant. Furthermore, differences were also observed in the percentage of animals that entered in *status epilepticus,* i.e., they presented tonic-clonic seizures lasting at least 30 minutes non-stop, within the NST0060+KA group, which is 29% (4 out of 14) with respect to the treatment regimen group treated with vehicle+KA, which is 79% (11 out of 14), demonstrating that compound NST0060 is antiepileptic in addition to anticonvulsant.

| | Convulsions | ***Status epilepticus*** |
|---|---|---|
| **KA+Vehicle** | **93%** | **79%** |
| **KA+NST0060** | **36%** | **29%** |

Due to these results, the inventors decided to investigate if the antiepileptic effect of NST0060 was accompanied by a delay in the latency period (time when the first convulsions occurred). As shown in Figure 18, differences were observed in the latency between both groups, being above 100 minutes in the NST0060+KA group (100.1$\pm$7.3 minutes), and this being greater in a statistically significant manner ($p<0.05$) than with the vehicle+KA treatment regimen (45.7$\pm$7.5 minutes), which confirms the antiepileptic and anticonvulsant properties of compound NST0060.

Based on these results, the inventors decided to investigate if the antiepileptic and anticonvulsant effect was confirmed with the levels of epilepsy and by the severity of the symptoms observed for two hours after inoculation of KA. As shown in Figure 19, the animals in the group with the vehicle+KA regimen reached levels of epilepsy above 4 on the Racine scale at several points in time, and especially after 40 minutes, whereas the NST0060+KA regimen did not cause at any time a mean level of epilepsy greater than 3 on said scale. In fact, the onset kinetics and severity of

the epileptogenic symptoms caused by KA are different in a statistically significant manner between treatment with NST0060 and with vehicle (F (1.26) = 6.8016; p = 0.0149; general analysis of variance by means of the ANOVA test with a Newman-Keuls post-hoc test).

These results definitively show the antiepileptic and anticonvulsant capacity of compound NST0060 with respect to the administration of an excitotoxic substance.

EXAMPLE 9

*In vivo* antioxidant effect of NST0060 with respect to the oxidative stress of the brain induced by an excitotoxic substance

Knowing the antiepileptic and anticonvulsant capacity of compound NST0060 against the action of an excitotoxic substance, the inventors decided to investigate if this neuroprotective effect shown by NST0060 with respect to excitotoxicity was accompanied by a decrease in the oxidative stress occurring in the brain when an excitotoxic substance is administered in mice.

All the animals included during the experimental process were 12-week old males from the FVB/N strain who were carriers of a gene construct with the promoter of a gene that could be modulated by oxidative stress bound to the firefly luciferase reporter gene. The experiments were carried out strictly complying with the standard entitled Guidance on the Operation of Animals (Scientific Procedures, Act. 1986*).* The animals were subjected to their respective quarantine period and were treated with maximum precaution to minimize possible contaminations during inoculations and handling.

The animals were intraperitoneally inoculated with 25 mg/kg of kainate (KA) dissolved in PBS. Seven mice were pretreated at 24 and 0.5 hours before inoculation with KA and subsequently on a daily basis up to 7 days post-inoculation by means of intraperitoneal injection with 0.25% methyl cellulose in PBS as a vehicle (vehicle+KA administration regimen), and another 7 mice were pretreated at 24 and 0.5 hours before inoculation with KA and subsequently on a daily basis up to 7 days post-inoculation by means of intraperitoneal injection with compound NST0060 at a dose of 50 mg/kg (NST0060+KA administration regimen). After inoculation, the animals were individually housed in trays to monitor them. Additionally, 3 mice were treated with the vehicle alone in all the administrations and they did not receive a KA injection so that they could act as controls. Oxidative stress in the brain was monitored by means of the *in vivo* bioluminescence technique with the IVIS Lumina system (Caliper LS). Acquisitions were taken at -1 dpi (baseline signal) and at 1, 2, 3 and 4 dpi in all the experimental groups. Prior to acquisition, the animals were inoculated intraperitoneally with D-luciferin at 150 mg/Kg. The mice were then anesthetised with 3% isofluorane for 2 minutes. Once housed in the IVIS Lumina system, the mice remained anesthetized with 1.5% isofluorane, $O_2$ at 0.2 L/min/mouse and compressed air at 2 L/min/mouse during the image acquisition period. The photon emission images were taken 15 minutes after the administration of D-luciferin with an acquisition period of 5 minutes at a highly sensitive position.

As shown in Figure 20, where images of the photon emission kinetics of the different experimental groups are shown, the administration of KA induces a significant increase in oxidative stress in the brain from 1 dpi up to 4 dpi, time point in which a greater increase in gene expression in the brain was recorded. Surprisingly, the animals inoculated with KA and treated with NST0060 did not show a significant increase in the expression of this gene associated with oxidative stress throughout the entire kinetics studied.

As shown in Figure 21, where the quantification of the mean signal of all the subjects corresponding to the different experimental groups is presented, the administration of KA induces a significant increase in oxidative stress in the brain from 1 dpi up to 4 dpi, time point in which a greater increase in gene expression in the brain was recorded. Surprisingly, the animals inoculated with KA and treated with NST0060 did not show a significant increase in the expression of this gene associated with oxidative stress throughout the entire kinetics studied.

These results indicate that compound NST0060 has antioxidant properties and that in the case of the brain, it protects against oxidative stress caused by the administration of an excitotoxic substance.

EXAMPLE 10

Protective effect of NST0060 against death caused by the acute administration of an excitotoxic substance in mice

Knowing the antiepileptic, anticonvulsant and antioxidant capacity of compound NST0060 against the action of an excitotoxic substance in mice, the inventors decided to investigate if this protective effect shown by NST0060 with respect to excitotoxicity was accompanied by a decrease in the levels of death caused by an excitotoxic substance in mice.

All the animals included during the experimental process were 12-week old males from the FVB/NHan strain. The experiments were carried out strictly complying with the standard entitled Guidance on the Operation of Animals (Scientific Procedures, Act. 1986*).* The animals were subjected to their respective quarantine period and were treated with maximum precaution to minimize possible contaminations during inoculations and handling.

The animals were intraperitoneally inoculated with 25 mg/kg of kainate (KA) dissolved in PBS. Fourteen animals

were pretreated at 24 and 0.5 hours before inoculation with KA and subsequently on a daily basis up to 7 days post-inoculation by means of intraperitoneal injection with 0.25% methyl cellulose in PBS as a vehicle (vehicle+KA administration regimen) and 14 were pretreated at 24 and 0.5 hours before inoculation with KA and subsequently on a daily basis up to 7 days post-inoculation by means of intraperitoneal injection with compound NST0060 at a dose of 50 mg/kg (NST0060+KA administration regimen). After inoculation, the animals were individually housed in trays to monitor them. Additionally, 8 mice were treated with the vehicle alone in all the administrations and they did not receive a KA injection so that they could act as controls. Deaths in the different treatment groups were recorded throughout the entire experiment, and this data was used to establish the corresponding survival curves that are shown in Figure 22.

The results indicated that the administration of a dose of 25 mg/Kg of intraperitoneal KA causes a statistically significant increase in death of the mice. Treatment with NST0060 before and after inoculation of KA surprisingly reduces mortality observed in a statistically significant manner ($p<0.05$).

These results indicate that compound NST0060 increases survival with respect to mortality caused by an excitotoxic substance.

## Claims

1. A compound of formula (I)

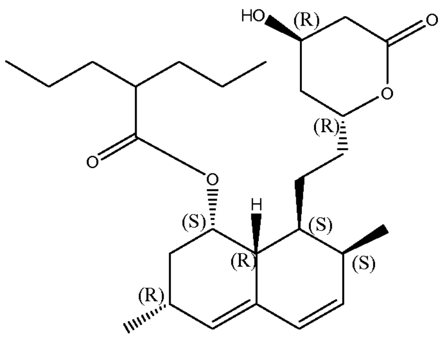

(I)

its hydroxy acid form, the pharmaceutically acceptable salts of said hydroxy acid and pharmaceutically acceptable prodrugs and solvates of the compound and of its hydroxy acid form.

2. A pharmaceutical composition comprising a compound of formula (I) according to claim 1 and/or its hydroxy acid form and/or a pharmaceutically acceptable salt of said hydroxy acid and/or a pharmaceutically acceptable prodrug or solvate of the compound or of its hydroxy acid form, and at least one pharmaceutically acceptable adjuvant, carrier and/or vehicle.

3. A compound of formula (I) according to claim 1, its hydroxy acid form or a pharmaceutically acceptable salt of said hydroxy acid and/or a pharmaceutically acceptable prodrug or solvate of the compound or of its hydroxy acid form for use as a medicinal product.

4. A compound of formula (I) according to claim 1, its hydroxy acid form or a pharmaceutically acceptable salt of said hydroxy acid and/or a pharmaceutically acceptable prodrug or solvate of the compound or of its hydroxy acid form for use in the prevention and/or the treatment of:

   a. neurodegenerative diseases,
   b. cognitive impairment,
   c. diseases associated with undesired oxidation,
   d. age-associated pathological processes and progeria,
   e. cardiovascular diseases such as atherosclerosis, atrial fibrillation, dyslipidemia, hypercholesterolemia, hyperlipidemia, and hypertriglyceridemia, or
   f. inflammation or inflammatory processes,

g. epilepsy, epileptic seizures and convulsions.

**5.** A compound of formula (I), its hydroxy acid form or a pharmaceutically acceptable salt of said hydroxy acid and/or a pharmaceutically acceptable prodrug or solvate of the compound or of its hydroxy acid form according to claim 4, wherein the neurodegenerative diseases are: Alzheimer's disease, Huntington's disease, Parkinson's disease, amyotrophic lateral sclerosis (ALS) or multiple sclerosis.

**6.** Use of a compound of formula (I) according to claim 1, of its hydroxy acid form, of a pharmaceutically acceptable salt of said hydroxy acid and/or of a pharmaceutically acceptable prodrug or solvate of the compound or of its hydroxy acid form in the preparation of a medicament.

**7.** Use according to claim 6, wherein the medicament is used in the prevention and/or the treatment of:

a. neurodegenerative diseases,
b. cognitive impairment,
c. diseases associated with undesired oxidation,
d. age-associated pathological processes and progeria,
e. cardiovascular diseases such as atherosclerosis, atrial fibrillation, dyslipidemia, hypercholesterolemia, hyperlipidemia, and hypertriglyceridemia, or
f. inflammation or inflammatory processes,
g. epilepsy, epileptic seizures and convulsions.

**8.** A compound of formula (I) according to claim 1, its hydroxy acid form or a pharmaceutically acceptable salt of said hydroxy acid and/or a pharmaceutically acceptable prodrug or solvate of the compound or of its hydroxy acid form for use in increasing seladin-1/DHCR24 gene expression.

**9.** A compound of formula (I) according to claim 1, its hydroxy acid form or a pharmaceutically acceptable salt of said hydroxy acid and/or a pharmaceutically acceptable prodrug or solvate of the compound or of its hydroxy acid form for use in the prevention and/or treatment of diseases related to the seladin-1/DHCR24 gene.

**10.** A compound of formula (I), its hydroxy acid form or a pharmaceutically acceptable salt of said hydroxy acid and/or a pharmaceutically acceptable prodrug or solvate of the compound or of its hydroxy acid form according to claim 9, wherein neuronal death associated with neurodegenerative diseases, diseases associated with undesired oxidation or age-associated pathological processes are prevented and/or treated.

**11.** Use of a compound of formula (I) according to claim 1, of its hydroxy acid form, of a pharmaceutically acceptable salt of said hydroxy acid and/or of a pharmaceutically acceptable prodrug or solvate of the compound or of its hydroxy acid form in the preparation of a medicament **characterized by** increasing seladin-1/DHCR24 gene expression.

**12.** Use of a compound of formula (I) according to claim 1, of its hydroxy acid form, of a pharmaceutically acceptable salt of said hydroxy acid and/or of a pharmaceutically acceptable prodrug or solvate of the compound or of its hydroxy acid form in the preparation of a medicament for the prevention and/or treatment of diseases related to the seladin-1/DHCR24 gene.

Figure 1

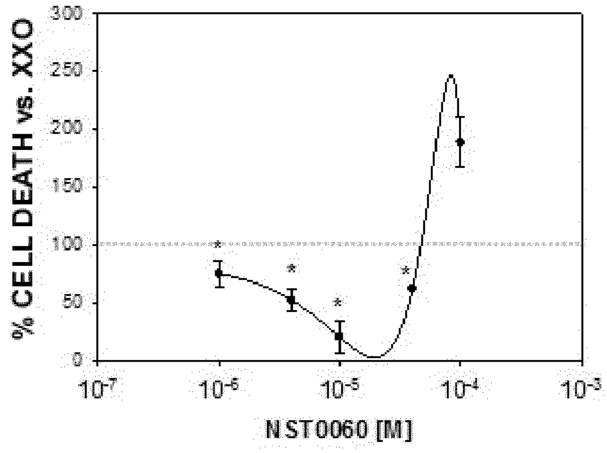


Figure 2

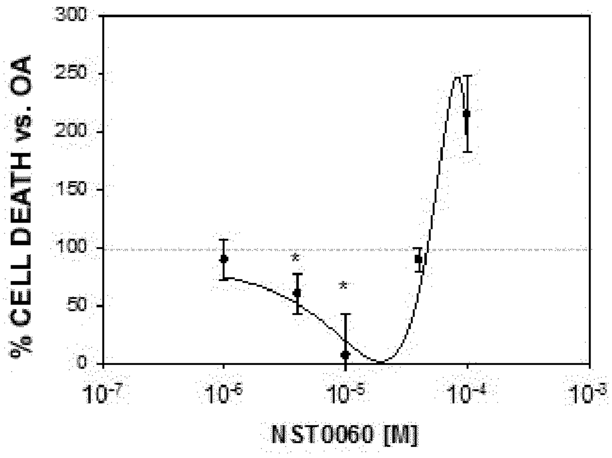

Figure 3

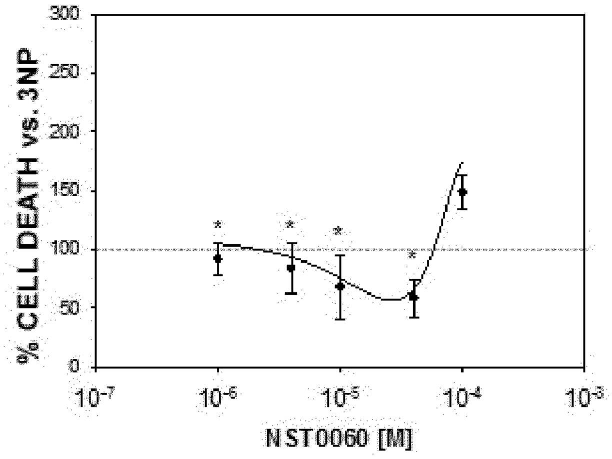


Figure 4

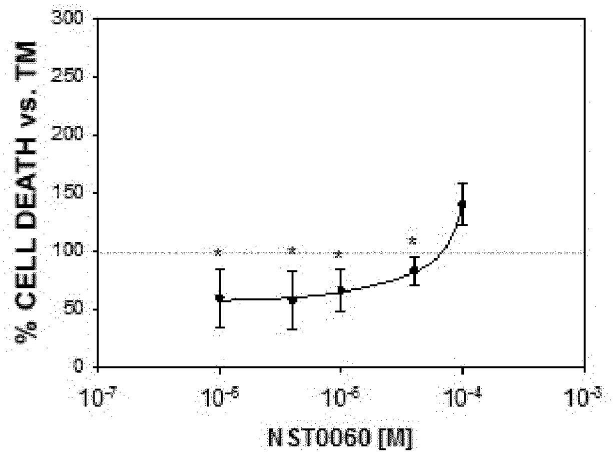

Figure 5

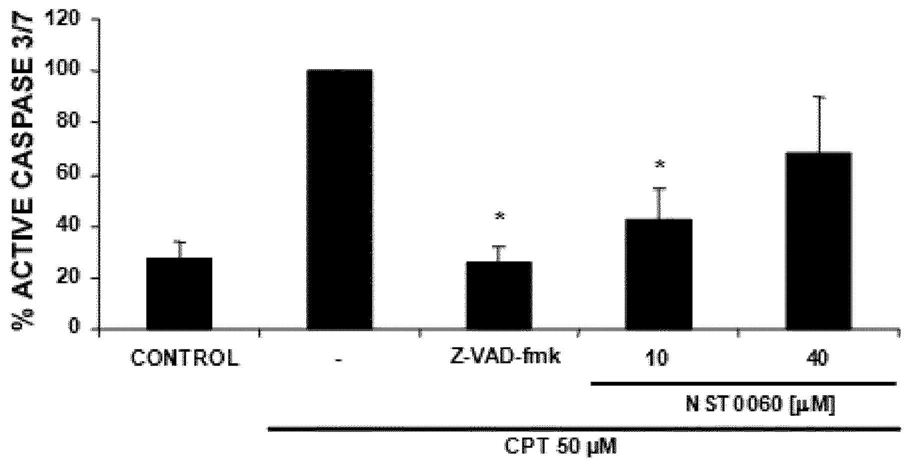


Figure 6

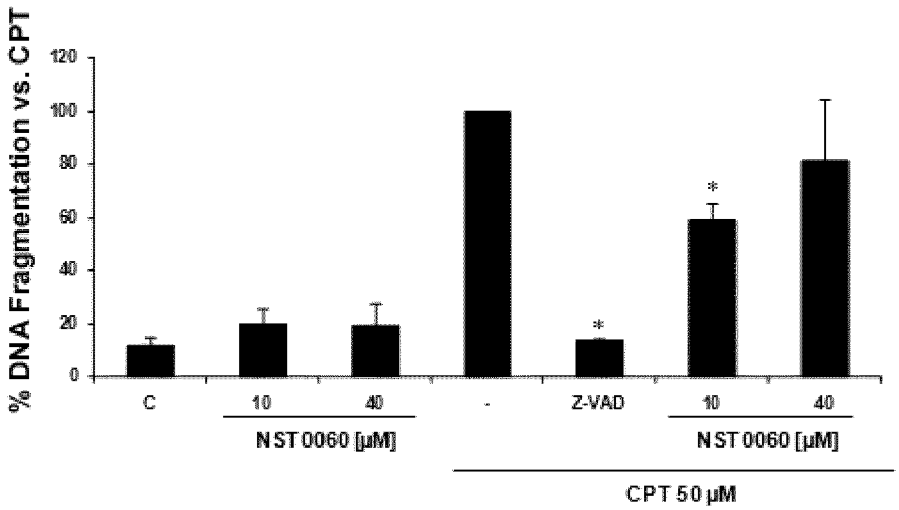

Figure 7

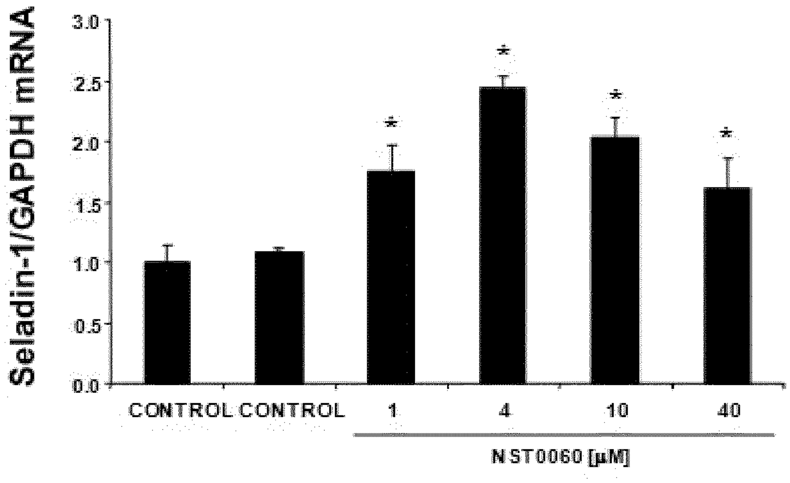


Figure 8

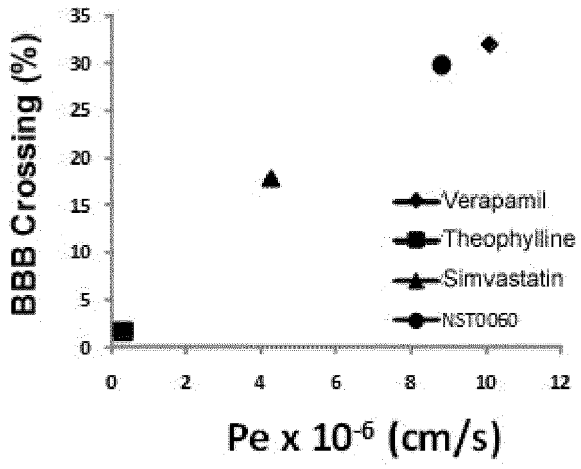

## Figure 9

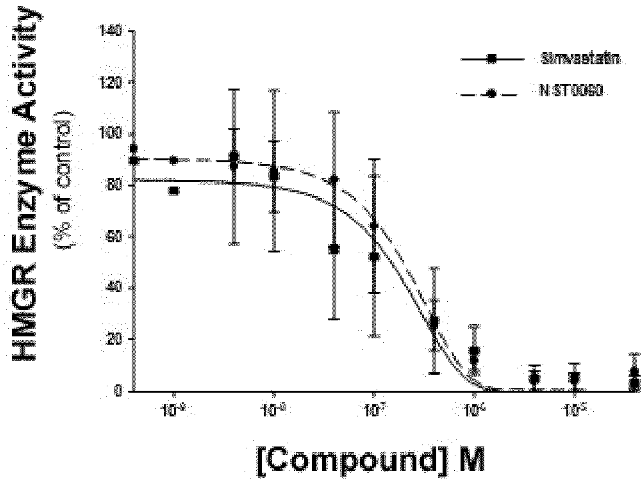


## Figure 10

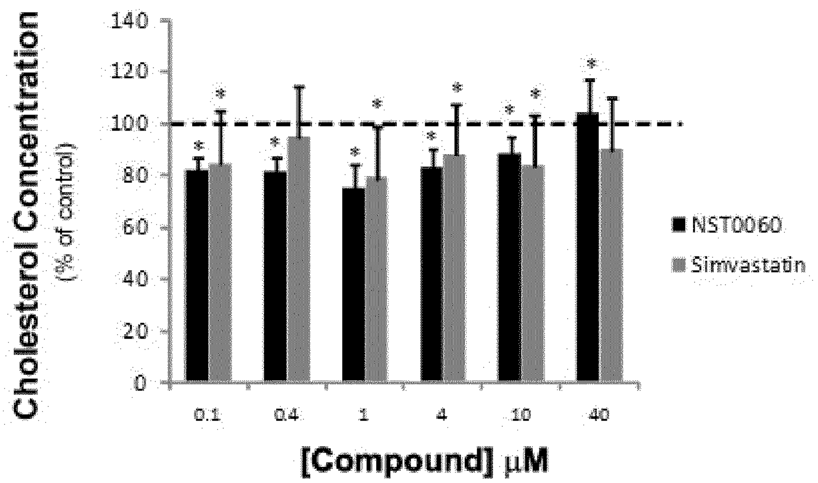

Figure 11

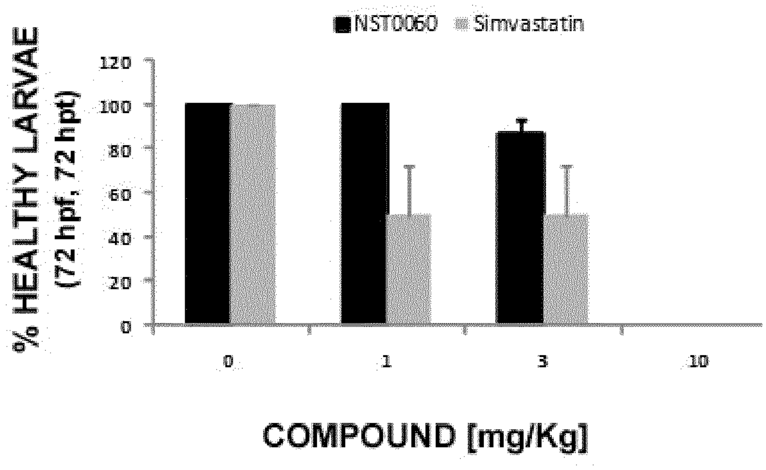


Figure 12

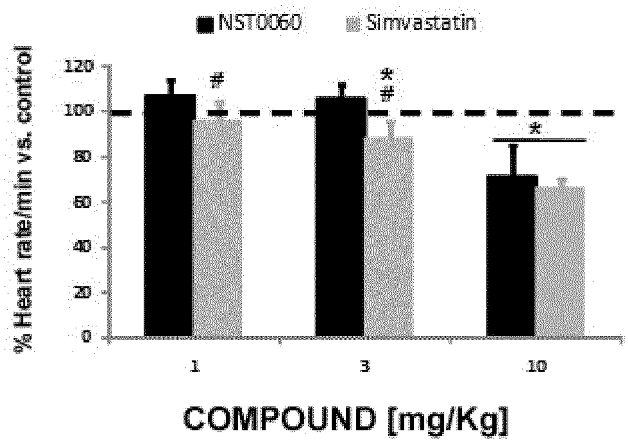

Figure 13

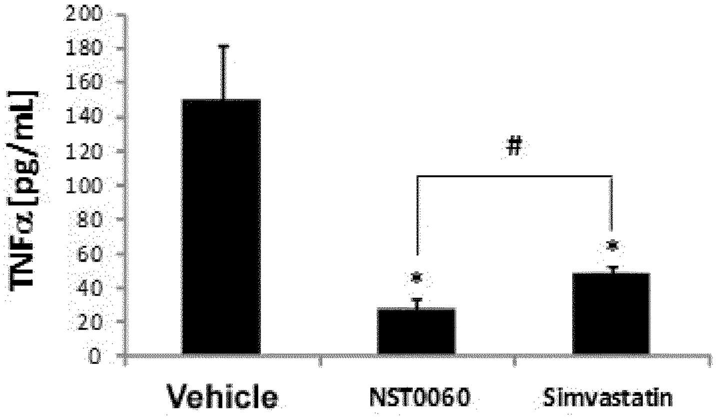


Figure 14

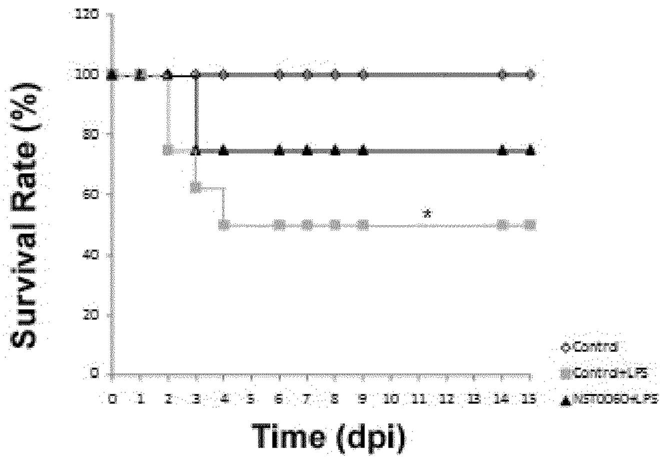

Figure 15

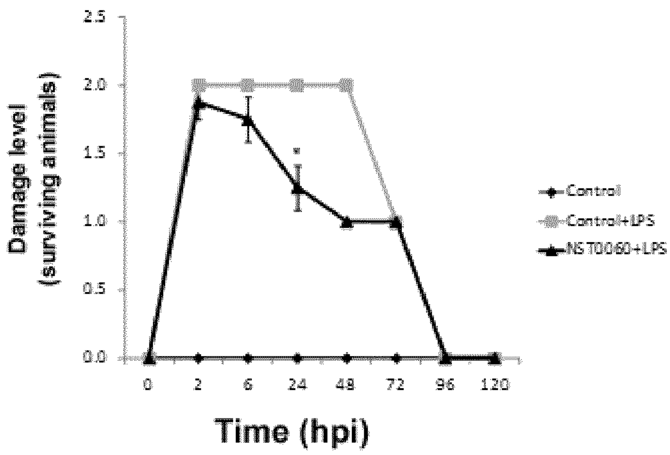


Figure 16

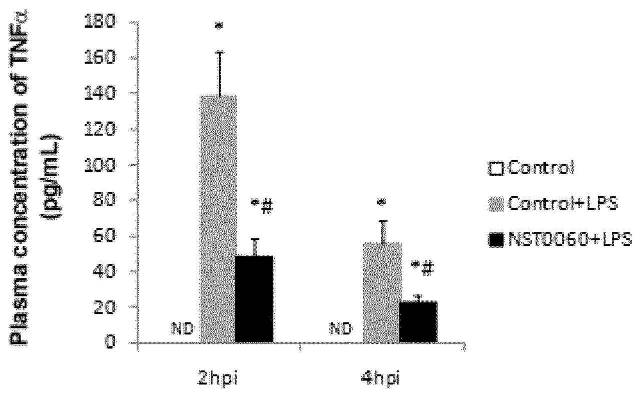

Figure 17

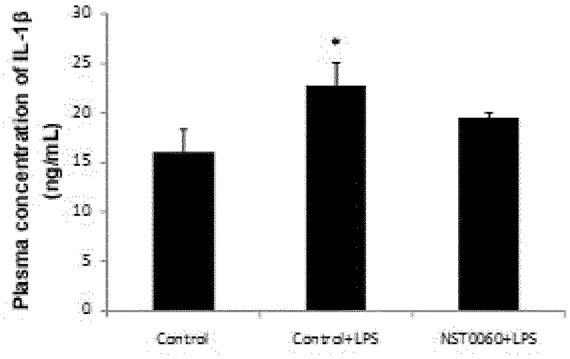


Figure 18

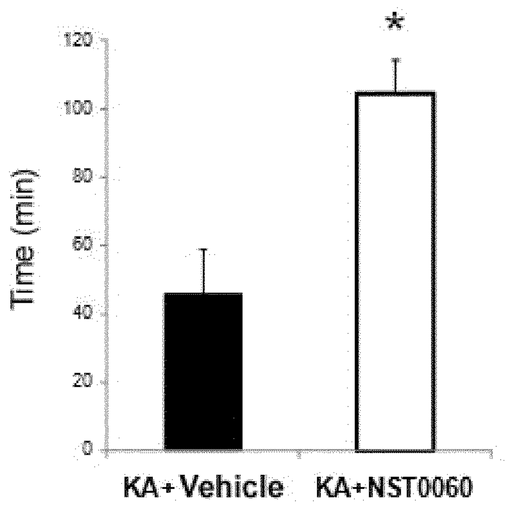

Figure 19

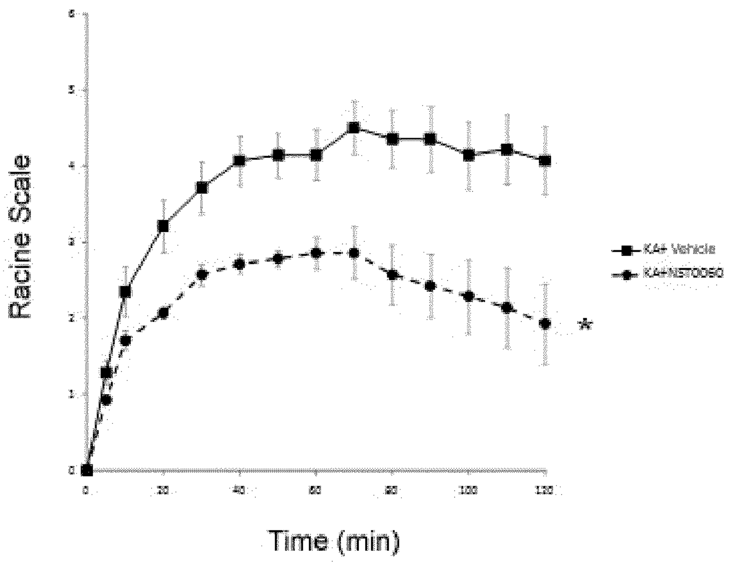


Figure 20

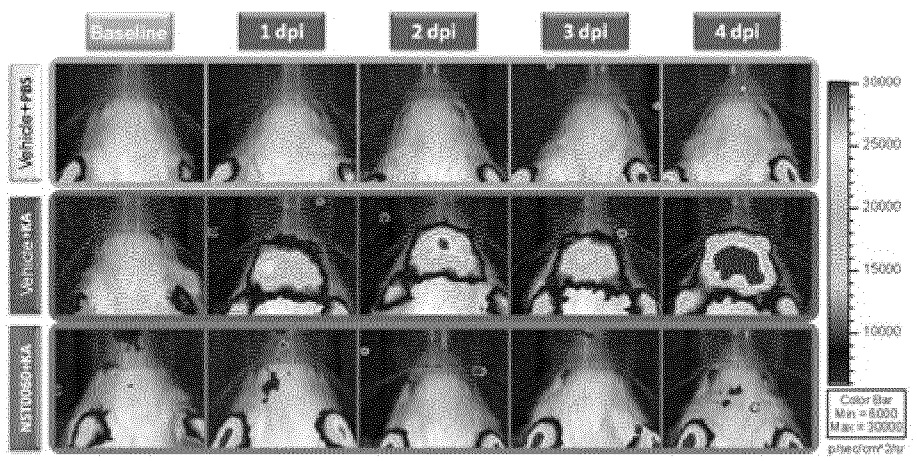

Figure 21

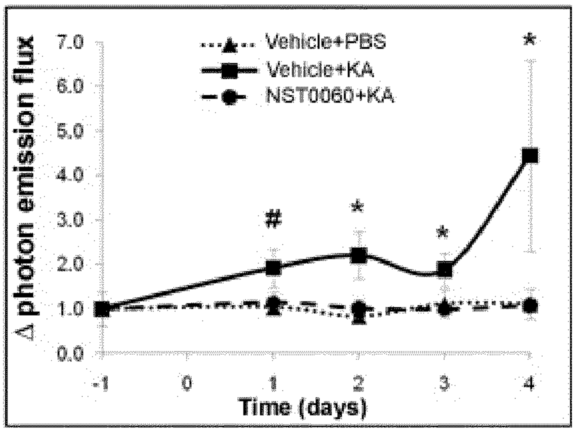


Figure 22

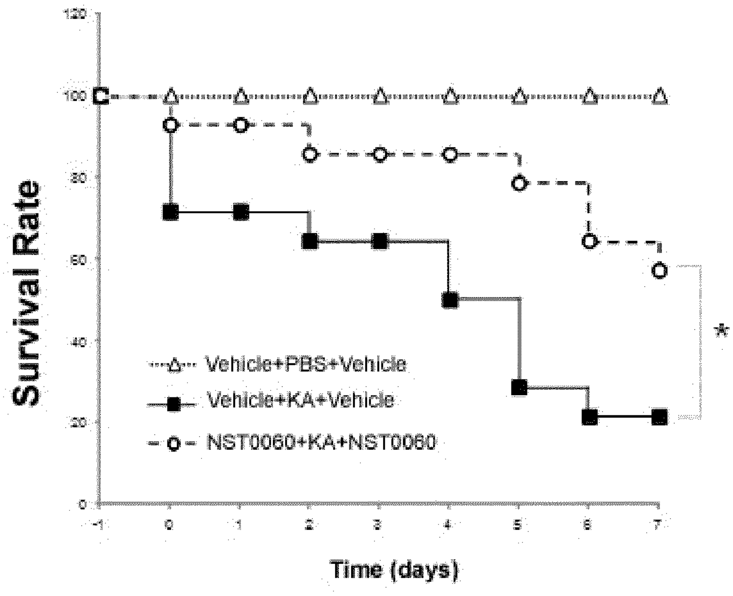

## INTERNATIONAL SEARCH REPORT

International application No.
PCT/ES2011/070705

A. CLASSIFICATION OF SUBJECT MATTER

**See extra sheet**

According to International Patent Classification (IPC) or to both national classification and IPC

B. FIELDS SEARCHED

Minimum documentation searched (classification system followed by classification symbols)
C07D, A61K, A61P

Documentation searched other than minimum documentation to the extent that such documents are included in the fields searched

Electronic data base consulted during the international search (name of data base and, where practicable, search terms used)

EPODOC, INVENES, WPI, XPESP, REGISTRY, HCAPLUS, MEDLINE, BIOSIS, NPL

C. DOCUMENTS CONSIDERED TO BE RELEVANT

| Category* | Citation of document, with indication, where appropriate, of the relevant passages | Relevant to claim No. |
|---|---|---|
| A | WO 9911258 A1 (NOVARTIS- ERFINDUNGEN VERWALTUNGSGESELLSCHAFT MBH) 11.03.1999, page 9, paragraph 3, page 16, claims 1, 4 | 1-12 |
| A | US 4450171 A (HOFFMAN ET AL) 22.05.1984, column 12, lines 45-65, reivinidicaciones, compuestos IIIa-e, tables | 1-12 |
| A | US 4293496 A (WILLARD ALVIN K) 06.10.1981, column 15, line 62-column 16, line 15, compuestos Ia-e, claims. | 1-12 |
| A | WO 2007089787 A2 (BRYSON, I. ) 09.08.2007, page 1, lines 8-15, page 3, lines 12-20, claims | 1-12 |

☒ Further documents are listed in the continuation of Box C. ☒ See patent family annex.

* Special categories of cited documents:
"A" document defining the general state of the art which is not considered to be of particular relevance.
"E" earlier document but published on or after the international filing date
"L" document which may throw doubts on priority claim(s) or which is cited to establish the publication date of another citation or other special reason (as specified)
"O" document referring to an oral disclosure use, exhibition, or other means.
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other documents , such combination being obvious to a person skilled in the art
"&" document member of the same patent family

| Date of the actual completion of the international search | Date of mailing of the international search report |
|---|---|
| 14/02/2012 | **(24/02/2012)** |

| Name and mailing address of the ISA/ | Authorized officer |
|---|---|
| OFICINA ESPAÑOLA DE PATENTES Y MARCAS<br>Paseo de la Castellana, 75 - 28071 Madrid (España)<br>Facsimile No.: 91 349 53 04 | H. Aylagas Cancio<br>Telephone No. 91 3498563 |

Form PCT/ISA/210 (second sheet) (July 2009)

**INTERNATIONAL SEARCH REPORT**

International application No.
PCT/ES2011/070705

C (continuation). DOCUMENTS CONSIDERED TO BE RELEVANT

| Category * | Citation of documents, with indication, where appropriate, of the relevant passages | Relevant to claim No. |
|---|---|---|
| A | LEE, J.K. ET AL.:"Statin inhibits kainic acid-induced seizure and associated inflammation and hippocampal cell death". Neuroscience Letters, August 2008, vol. 440, nº 3, pages 260-264, page 260, page 261, primer paragraph. | 1-12 |
| A | VOLLMER, T. ET AL." Oral Simvastatin treatment in relapsing- 1-6 remitting multiple sclerosis". The Lancet. 15 May 2004, Vol. 363, pages 1607-1608, the whole document. | 1-12 |
| A | CORDLE, A ET Al.: "3-Hydroxy-3-Methylglutaryl-Coenzyme A 1-6 Reductase Inhibitors Attenuate Beta-Amyloid-Induced Microglial Inflammatory Responses". The Journal of Neuroscience. 2005, Vol. 25, Nº 2, pages 299-307, the whole document | 1-12 |
| A | WOLOZIN, B. ET AL. "Simvastatin is associated with a reduced 1-6 incidence of dementia and Parkinson's disease". BMC Medicine. 2007, Vol. 5, Nº 20, pages 1-11, the whole document. | 1-12 |

Form PCT/ISA/210 (continuation of second sheet) (July 2009)

**INTERNATIONAL SEARCH REPORT**

Information on patent family members

International application No.
PCT/ES2011/070705

| Patent document cited in the search report | Publication date | Patent family member(s) | Publication date |
|---|---|---|---|
| WO9911258 A | 11.03.1999 | ZA9807787 A | 01.03.1999 |
| | | AU9739198 A | 22.03.1999 |
| | | EP1007033 AB | 14.06.2000 |
| | | EP19980951317 | 26.08.1998 |
| | | CO4970739 A | 07.11.2000 |
| | | AU737735 B | 30.08.2001 |
| | | JP2001514221 A | 11.09.2001 |
| | | JP4340386B2 B | 07.10.2009 |
| | | NZ502872 A | 25.10.2002 |
| | | US6630492 B | 07.10.2003 |
| | | TW593297 B | 21.06.2004 |
| | | AT392894 T | 15.05.2008 |
| | | PT1007033 E | 22.07.2008 |
| | | ES2306482 T | 01.11.2008 |
| | | DE69839399 T | 20.05.2009 |
| US4450171 A | 22.05.1984 | PT72441 AB | 01.03.1981 |
| | | IE51478 B | 07.01.1987 |
| | | IE810204 L | 04.08.1981 |
| | | NO810358 A | 05.08.1981 |
| | | NO154229 B | 05.05.1986 |
| | | NO154229 C | 13.08.1986 |
| | | DK46181 A | 05.08.1981 |
| | | DK157292 B | 04.12.1989 |
| | | DK157292 C | 07.05.1990 |
| | | FI810287 A | 05.08.1981 |
| | | FI78082 B | 28.02.1989 |
| | | FI78082 C | 12.06.1989 |
| | | EP0033538 AB | 12.08.1981 |
| | | EP19810100729 | 02.02.1981 |
| | | MA19054 A | 01.10.1981 |
| | | DD155989 A | 21.07.1982 |
| | | YU28181 A | 31.10.1983 |
| | | PH16615 A | 28.11.1983 |
| | | RO82367 AB | 21.02.1984 |
| | | US4444784 A | 24.04.1984 |
| | | GR74798 A | 12.07.1984 |
| | | NZ196172 A | 31.01.1985 |
| | | NZ200588 A | 28.02.1985 |
| | | AT16704 T | 15.12.1985 |
| | | HU187296 B | 28.12.1985 |
| | | AU548996 B | 09.01.1986 |
| | | CA1199322 A | 14.01.1986 |
| | | ES8609296 A | 16.12.1986 |
| | | IL62044 A | 31.03.1987 |
| | | KE3746 A | 02.10.1987 |
| | | SG61087 G | 23.10.1987 |
| | | MY8700745 A | 31.12.1987 |
| | | HK16488 A | 11.03.1988 |
| | | CY1404 A | 22.04.1988 |

Form PCT/ISA/210 (patent family annex) (July 2009)

## INTERNATIONAL SEARCH REPORT

Information on patent family members

International application No.
PCT/ES2011/070705

| Patent document cited in the search report | Publication date | Patent family member(s) | Publication date |
|---|---|---|---|
| | | NL930009 I | 01.04.1993 |
| | | ECSP941119 A | 16.01.1995 |
| | | HR930775 A | 30.06.1997 |
| | | BG61418 B | 31.07.1997 |
| US4293496 A | 06.10.1981 | PT72441 AB | 01.03.1981 |
| | | IE51478 B | 07.01.1987 |
| | | IE810204 L | 04.08.1981 |
| | | NO810358 A | 05.08.1981 |
| | | NO154229 B | 05.05.1986 |
| | | NO154229 C | 13.08.1986 |
| | | DK46181 A | 05.08.1981 |
| | | DK157292 B | 04.12.1989 |
| | | DK157292 C | 07.05.1990 |
| | | FI810287 A | 05.08.1981 |
| | | FI78082 B | 28.02.1989 |
| | | FI78082 C | 12.06.1989 |
| | | EP0033538 AB | 12.08.1981 |
| | | EP19810100729 | 02.02.1981 |
| | | AU6657381 A | 13.08.1981 |
| | | JP56122375 A | 25.09.1981 |
| | | JP1001476 B | 11.01.1989 |
| | | JP1528833 C | 15.11.1989 |
| | | MA19054 A | 01.10.1981 |
| | | DD155989 A | 21.07.1982 |
| | | ZW2681 A | 01.09.1982 |
| | | ZA8100703 A | 29.09.1982 |
| | | PL229513 A | 28.03.1983 |
| | | PL131423 B | 30.11.1984 |
| | | PL237929 A | 28.03.1983 |
| | | PL133813 B | 29.06.1985 |
| | | YU28181 A | 31.10.1983 |
| | | PH16584 A | 22.11.1983 |
| | | RO82367 AB | 21.02.1984 |
| | | GR74798 A | 12.07.1984 |
| | | NZ196172 A | 31.01.1985 |
| | | NZ200588 A | 28.02.1985 |
| | | CS233743 B | 14.03.1985 |
| | | CS233718 B | 14.03.1985 |
| | | HUT34741 A | 28.04.1985 |
| | | KR850000669 B | 09.05.1985 |
| | | AT16704 T | 15.12.1985 |
| | | AU548996 B | 09.01.1986 |
| | | CA1199322 A | 14.01.1986 |
| | | ES8609296 A | 16.12.1986 |
| | | IL62044 A | 31.03.1987 |
| | | KE3746 A | 02.10.1987 |
| | | SG61087 G | 23.10.1987 |
| | | MY8700745 A | 31.12.1987 |

Form PCT/ISA/210 (patent family annex) (July 2009)

## INTERNATIONAL SEARCH REPORT

Information on patent family members

International application No.
PCT/ES2011/070705

| Patent document cited in the search report | Publication date | Patent family member(s) | Publication date |
|---|---|---|---|
| | | HK16488 A | 11.03.1988 |
| | | CY1404 A | 22.04.1988 |
| | | NL930009 I | 01.04.1993 |
| | | HR930775 A | 30.06.1997 |
| WO2007089787 A | 09.08.2007 | CA2640615 A | 09.08.2007 |
| | | AU2007209859 A | 09.08.2007 |
| | | US2007208073 A | 06.09.2007 |
| | | MX2008009591 A | 24.09.2008 |
| | | EP1984026 A | 29.10.2008 |
| | | EP20070717141 | 30.01.2007 |
| | | CN101374553 A | 25.02.2009 |
| | | JP2009525281 A | 09.07.2009 |
| | | RU2008135360 A | 10.03.2010 |
| | | BRPI0706757 A | 05.04.2011 |

Form PCT/ISA/210 (patent family annex) (July 2009)

## INTERNATIONAL SEARCH REPORT

International application No.

PCT/ES2011/070705

### CLASSIFICATION OF SUBJECT MATTER

***C07D309/30*** (2006.01)
***A61K31/366*** (2006.01)
***A61P25/28*** (2006.01)
***A61P25/08*** (2006.01)

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4866090 A


### Non-patent literature cited in the description


- **BLENNOW et al.** *Lancet,* 2006, vol. 368, 387-403
- **COLE ; VASSAR.** *Neurobiol Dis,* 2006, vol. 22 (2), 209-22
- **PAHAN.** *Cell Mol Life Sci.,* 2006, vol. 63 (10), 1165-78
- **LING et al.** *Ann Neurol.,* 2006, vol. 60 (6), 729-39
- **LEE et al.** *Neurosci Lett.,* 2008, vol. 440, 260-4
- **FANDINO et al.** *Neurocirugia,* 2007, vol. 18, 16-27
- **HONJO et al.** *Arch. Ophthalmol.,* 2002, vol. 120, 1707-13
- **HOLMES et al.** Systemic inflammation and disease progression in Alzheimer disease. *Neurology,* 2009
- **RACINE.** *Electroencephalogr Clin Neurophysiol,* 1972, vol. 32 (3), 281-94
- **CECHI et al.** *J. Cell. Mol. Med.,* 2008, vol. 12, 1990-2002
- **GREEVE et al.** *J. Neurosci.,* 2000, vol. 20, 7345-52
- **C. FAULÍ I TRILLO.** Treated de Farmacia Galénica. 1993
- **HOFFMAN et al.** *J. Med. Chem.,* 1986, vol. 29, 849-852
- Scientific Procedures, Act. Guidance on the Operation of Animals. 1986
- Scientific Procedures, Act. *Guidance on the Operation of Animals,* 1986